# EUROPEAN PATENT SPECIFICATION

(11) **EP 2 298 798 B1**
(45) Date of publication and mention of the grant of the patent: **09.05.2012**
(21) Application number: 10176013.0
(22) Date of filing: 09.09.2010
(51) Int. Cl.: C07K 14/435

(54) **Identification and characterization of a novel avian pathogenic E. coli (APEC) fimbrial adhesin**
Identifizierung und Charakterisierung eines neuartigen aviären pathogenen E. coli (APEC) Fimbrien-Adhäsins
L'identification et la caractérisation de la pathogène aviaire adhésine fimbriée (APEC) d'E. coli

(30) Priority: 10.09.2009 US 241069 P
(43) Date of publication of application: 23.03.2011
(73) Proprietor: Freie Universität Berlin, 14195 Berlin (DE); Lohmann Tierzucht GmbH, 27454 Cuxhaven (DE)
(72) Inventor: Antao, Esther-Maria, 14057 Berlin (DE); Ganwu, Li, Ames, IA 50010 (US); Wieler, Lothar, 14109 Berlin (DE); Preisinger, Rudolf, 21789 Wingst (DE); Ewers, Christa, 10827 Berlin (DE)
(74) Representative: Gulde Hengelhaupt Ziebig & Schneider

(56) References cited:
- WO-A2-01/66572
- WO-A2-2006/089264
- DATABASE UniProt [Online] 26 May 2009 (2009-05-26), "SubName: Full=Putative uncharacterized protein;", XP002617120, retrieved from EBI accession no. UNIPROT:C1HHU8 Database accession no. C1HHU8
- EWERS CHRISTA ET AL: "Intestine and Environment of the Chicken as Reservoirs for Extraintestinal Pathogenic Escherichia coli Strains with Zoonotic Potential", APPLIED AND ENVIRONMENTAL MICROBIOLOGY, vol. 75, no. 1, January 2009 (2009-01), pages 184-192, XP002617119, ISSN: 0099-2240
- ANTAO ESTHER-MARIA ET AL: "Signature-Tagged Mutagenesis in a Chicken Infection Model Leads to the Identification of a Novel Avian Pathogenic Escherichia coli Fimbrial Adhesin", PLOS ONE, vol. 4, no. 11, November 2009 (2009-11), XP002617118, ISSN: 1932-6203

### SEQUENCE LISTING

<110> Freie universität Berlin
<120> Identification and characterization of a Novel Avian Pathogenic E. coli (APEC) Fimbrial Adhesin
<130> p693709EP
<150> US 61/241,069
   <151> 2009-09-10
<160> 23
<170> PatentIn version 3.3
<210> 1
   <211> 1050
   <212> DNA
   <213> Escherichia coli strain IMT5155
<400> 1
   atgattacgc tttttcgttt actggcgatt ctttgcctgt tttttaacgt ttcagctttt 60
   gctgttgatt gctatcagga tgggtacaga ggaacaaccc tcataaatgg agatttacca 120
   gcgttcaaaa ttccagagaa cgcgcaacct gggcaaaaaa tttgggagag tggagatatt 180
   aatatcacgg tttattgtga caatgcacca ggatggacaa cagacaatcc atcagaaaat 240
   atctatgcct ggatcaaatt accccaaata aatagtgccg acatgttgaa taatccatat 300
   ttaacctttg gcgtgactta taatggtgta gattatgaag ggataaatga aagaattgat 360
   actcatgcat gcctggataa atatgagcaa tactataatg ggtattatta tgaccctgta 420
   tgcaatggca gtacccttca aaaaaatgta acatttaatg cccattttcg cgtctatgta 480
   aaattcaaaa gccgcccagc aggagatcag acggtgaact ttggcatagt caacgtgtta 540
   caattcgatg gcgaaggagg ggcaaacatg tcctctaacg cgaagaattt acgctatgtg 600
   attacgggat tagataatat ttcattcctt gactgtagtg tcgacgtccg gatttttcca 660
   gaaagccaga tagttaattt cgggcaaatt gctgcaaact ctattgctac ttatcgacca 720
   aaagctgcat ttagcgtatc tactataaaa gatgttgctg ccgattgtac cgagcaattt 780
   gatgttgcca ccagctttta tacgacagat acgttacatg atgatacgca tctggaaatg 840
   ggtaatggtt tgttgatgcg tattactgat caaaaaacac aagaagacat taagtttaat 900
   cagtacaaac gcttcaccac atatattcca ggtcagactg gagcgatggt tacccgtgat 960
   taccaggctg aactttcaca aaaaccgggg gagactctcg tttatgggcc tttcaaaaaa 1020
   gatctcatcg taaaaataaa ctataactga 1050
<210> 2
   <211> 4975
   <212> DNA
   <213> Escherichia coli strain IMT5155
<400> 2
   atgttaaaaa aaacattgtt atctatgttc gcaaccgcat tgttatcagg cgttgctttt 60
   aacgctcttg ctgacgatgc taatcagggt tcaggtaaaa ttacttttaa aggtgaagtt 120
   atcgatgcac cttgttctat tgctcctggt gatgaagatc agacaataaa cctcggtgaa 180
   gttgctgata ccgtattaaa aagcggtcag aaatcactgc ctgtagatgc caccattcat 240
   ttgcaggatt gtattttatc tgacggcact aacactgttg ataaagtcaa aatcaccttt 300
   agttctgcca gtgttgacgc taccgactcc aacctgctta aaaacactct ggaaggtaac 360
   atcggcggcg caactgatgt aggcgtacgt ctggtgaaat cagacaacac caacgtgact 420
   cttggcactc caatcactat caacttcccg acgactagct cttaccagga gttgaacttt 480
   aaagcccgta tggagtctct gggacgcacc gcgaccccgg gtaacgtgca ggcacaggct 540
   aattacgtac tcgactacaa gtaatcggta gtcaatacct acacaacgcc aggcggtaaa 600
   gccgatgaaa tttcatcggc aactttgggc ctttttagaa atggattttt atgaacaata 660
   aaaacacgtt ttcccgggat aagttatccc atgcaattaa aaatgccctg tctggcgttg 720
   tgtgttccct actcttcgtt ttgccagtcc acgccgtaga attcaacgtc gatatgattg 780
   acgcagaaga ccgtgagaat atcgacatct ctcgttttga gaaaaaaggc tatatccccc 840
   ctggtagata cctcgttcgt gtgcaaataa ataaaaatat gttgccacaa acgttaatac 900
   tggaatgggt aaaagccgat aatgaaagtg gttcgttact ctgcttaacc aaagaaaatt 960
   tgactaattt cggtcttaat acggaattta ttgaatcatt gcaaaacata gctggcagcg 1020
   aatgtctcga tttaagccaa cgtcaggagt taacgacacg acttgataaa gctacgatga 1080
   tattatcgct aagtgttccc caggcatggt taaaatacca ggcaacaaac tggacgccac 1140
   cagagttttg ggataccggt atcgccgggt ttatccttga ttacaacgtg tacgccagcc 1200
   agtatgcccc acatcacgga gacagcaccc aaaacgtcag ctcctatggt acgttaggct 1260
   ttaacctcgg cgcatggcgc ttacgtagcg attaccaata taatcagaat tttgctgatg 1320
   gacgctcggt aaaccgcgac agcgaatttg cgcgaactta tctgtttcgc cctatcccct 1380
   cctggtcgtc aaaattcact atgggccagt acgacctgag ctccaatctt tacgatacct 1440
   tccactttac tggcgcatcg ctggaaagtg atgaaagcat gctgccgcca gatttacagg 1500
   gttatgcgcc acaaattacc ggcatcgcgc agaccaacgc gaaagtaact gtggcacaaa 1560
   atggtcgtgt actttatcaa accactgtcg cgccaggccc ttttactatt tctgatttgg 1620
   ggcaatcgtt tcaggggctg ctggatgtca cagtggaaga agaagatggc cgcaccagca 1680
   ccttccaggt tggctccgca tccattccct atttaacccg taaagggcaa gtgcgctata 1740
   aaacgtcact gggaaaaccg acatccgtcg ggcataacga tatcaataat ccctttttct 1800
   ggacggcgga agcctcctgg ggctggctga acaatgtgtc gttgtatggt ggtggcatgt 1860
   tcaccgctga tgattatcag gctatcacta ccggtattgg ctttaacctt aaccaattcg 1920
   gttcgctttc ttttgatgtc actggagcag acgcgtcttt acagcaacaa aatagcggca 1980
   atctgcgtgg ttacagctat cgcttcaact atgcaaagca tttcgaatcg acaggcagtc 2040
   agattacctt cgcgggttat cgcttctcag ataaagatta cgtgtcgatg agtgggtacc 2100
   tcagctcgcg taatggcgat gagtcaaccg ataatgaaaa agagagttat gtcatttcct 2160
   tgaaccagta ctttgaaacg ctggaattaa actcttatct caacgttaca cgcaatactt 2220
   attgggacag cgccagcaat accaactact ccgtatctgt aagcaaaaac tttgatattg 2280
   gcgatttcaa aggtatatct gcatcgctgg cagtaagtcg aatccgctgg gatgacgacg 2340
   aagagaatca atattacttc tctttctctc tacctttaca acaaaaccgc aacatctcct 2400
   acagtatgca gcgaacggga agcagtaata cttcgcagat gatttcctgg tacgattcat 2460
   cagatcgcaa caatatctgg aatatttcag cgtcggcagc ggacgacaat atacgtgatg 2520
   gcgaaccaac actgcgcggc agctaccagc actattcgcc gtggggacgt ctgaacatta 2580
   atggcagtgt acagccgaat cagtacaatt ctgttaccgc aggctggtac ggttcactta 2640
   ccgctacacg tcatggtatc gcccttcacg attatagcta tggcgataac gcccgcatga 2700
   tggtcgatac cgatggcatc tccggcattg aaatcaactc taaccgtacc gttaccaacg 2760
   ggctgggcat cgccgtgata ccttcgttat cgaactacac cacctccatg ttgcgggtga 2820
   acaataacga tctgccagaa ggtgtcgatg tcgaaaactc ggttattcgc accacgctca 2880
   cccagggtgc catcggctac gcaaaactga atgccaccac cggttaccaa atcgtcggcg 2940
   ttattcgtca ggaaaatggg cgcttccctc cactaggtgt gaatgtcacg gataaagcga 3000
   caggtaaaga tgtgggcctg gtcgcggaag acggcttcgt ttatctcagc ggtattcagg 3060
   aaaatagtac tctgcattta acctggggtg ataatacctg tgaagtcacg ccgccaaacc 3120
   aaagtaacat tagcgaaagc gcaataattt taccttgtaa aacagtcaaa taattcagga 3180
   tgaatataat gaaatcacca ttaatcacaa caggaatgat ttgtttactg gggatctgca 3240
   actttgccca ggcgactgtc tctcccgaca gaacacgcat catttttaat gcttcgaata 3300
   aaagcgcaac ggttcgcctg accaaccaaa gtaaaatcga tccctatctg gctcagtcgt 3360
   ggattgaaga tgccagcggt aaaaaaacgc gtgattacat ctcgacattg ccgccgatgg 3420
   aacgtattga gcctgatgaa cagatacaaa ttcgcctgat ggcactggcc tcgttaaacg 3480
   acttaccaca ggaccgagaa acgttgttct actataacgt gcgagaaatt ccgccgcgcg 3540
   ccaaagaaca gaatgtaatg caaatcgcca tgcaaagccg cctgaagcta ttctggcggc 3600
   caaaagcgat tgagttaaaa gagggggaga tgattcctct gcaaaaagta acgattaccc 3660
   gcaccgccgc cggtctgacc ctgaataacc cgacgcctca tcatattacc gtcggttata 3720
   tcggcactaa tggcaagaca ctgatgcccg gtgcggacag tattatggtg gtaccattta 3780
   cttctgcaac tcaacaccta agcagcctgc cctctacctt ccagttaggc tttgtggcgg 3840
   actacggcgg gctggagatg tttaaagtcg aatgtaattc catccaatcc ctctgccaaa 3900
   gctctcctgc taagaagggg aaaatatgat tacgcttttt cgtttactgg cgattctttg 3960
   cctgtttttt aacgtttcag cttttgctgt tgattgctat caggatgggt acagaggaac 4020
   aaccctcata aatggagatt taccagcgtt caaaattcca gagaacgcgc aacctgggca 4080
   aaaaatttgg gagagtggag atattaatat cacggtttat tgtgacaatg caccaggatg 4140
   gacaacagac aatccatcag aaaatatcta tgcctggatc aaattacccc aaataaatag 4200
   tgccgacatg ttgaataatc catatttaac ctttggcgtg acttataatg gtgtagatta   4260
   tgaagggata aatgaaagaa ttgatactca tgcatgcctg gataaatatg agcaatacta   4320
   taatgggtat tattatgacc ctgtatgcaa tggcagtacc cttcaaaaaa atgtaacatt   4380
   taatgcccat tttcgcgtct atgtaaaatt caaaagccgc ccagcaggag atcagacggt   4440
   gagctttggc atagtcaacg tgttacaatt cgatggcgaa ggaggggcaa acatgtcctc   4500
   taacgcgaag aatttacgct atgtgattac gggattagat gatatttcat tccttgactg   4560
   tagtgtcgac gtccggattt ttccagaaag ccagatagtt aatttcgggc aaattgctgc   4620
   aaactctatt gctacttatc gaccaaaagc tgcatttagc gtatctacta taaaagatgt   4680
   tgctgccgat tgtaccgagc aatttgatgt tgccaccagc ttttatacga cagatacgtt   4740
   acatgatgat acgcatctgg aaatgggtaa tggttttttg atgcgtatta ctgatcaaaa   4800
   aacacaagaa gacattaagt ttaatcagta caaacgcttc accacatata ttccaggtca   4860
   gactggagcg atggttaccc gtgattacca ggctgaactt tcacaaaaac cgggggagac   4920
   tctcgtttat gggcctttca aaaaagatct catcgtaaaa ataaactata actga        4975
<210> 3
   <211> 349
   <212> PRT
   <213> Escherichia coli strain IMT5155
<400> 3
   Met Ile Thr Leu Phe Arg Leu Leu Ala Ile Leu Cys Leu Phe Phe Asn
   1               5                   10                  15
   Val Ser Ala Phe Ala Val Asp Cys Tyr Gln Asp Gly Tyr Arg Gly Thr
               20                  25                  30
   Thr Leu Ile Asn Gly Asp Leu Pro Ala Phe Lys Ile Pro Glu Asn Ala
           35                  40                  45
   Gln Pro Gly Gln Lys Ile Trp Glu Ser Gly Asp Ile Asn Ile Thr Val
       50                  55                  60
   Tyr Cys Asp Asn Ala Pro Gly Trp Thr Thr Asp Asn Pro Ser Glu Asn
   65                  70                  75                  80
   Ile Tyr Ala Trp Ile Lys Leu Pro Gln Ile Asn Ser Ala Asp Met Leu
                   85                  90                  95
   Asn Asn Pro Tyr Leu Thr Phe Gly Val Thr Tyr Asn Gly Val Asp Tyr
               100                 105                 110
   Glu Gly Ile Asn Glu Arg Ile Asp Thr His Ala Cys Leu Asp Lys Tyr
           115                 120                 125
   Glu Gln Tyr Tyr Asn Gly Tyr Tyr Tyr Asp Pro Val Cys Asn Gly Ser
   130 135 140
   Thr Leu Gln Lys Asn Val Thr Phe Asn Ala His Phe Arg Val Tyr Val
   145 150 155 160
   Lys Phe Lys Ser Arg Pro Ala Gly Asp Gln Thr Val Asn Phe Gly Ile
   165 170 175
   Val Asn Val Leu Gln Phe Asp Gly Glu Gly Gly Ala Asn Met Ser Ser
   180 185 190
   Asn Ala Lys Asn Leu Arg Tyr Val Ile Thr Gly Leu Asp Asn Ile Ser
   195 200 205
   Phe Leu Asp Cys Ser Val Asp Val Arg Ile Phe Pro Glu Ser Gln Ile
   210 215 220
   Val Asn Phe Gly Gln Ile Ala Ala Asn Ser Ile Ala Thr Tyr Arg Pro
   225 230 235 240
   Lys Ala Ala Phe Ser Val Ser Thr Ile Lys Asp Val Ala Ala Asp Cys
   245 250 255
   Thr Glu Gln Phe Asp Val Ala Thr Ser Phe Tyr Thr Thr Asp Thr Leu
   260 265 270
   His Asp Asp Thr His Leu Glu Met Gly Asn Gly Leu Leu Met Arg Ile
   275 280 285
   Thr Asp Gln Lys Thr Gln Glu Asp Ile Lys Phe Asn Gln Tyr Lys Arg
   290 295 300
   Phe Thr Thr Tyr Ile Pro Gly Gln Thr Gly Ala Met Val Thr Arg Asp
   305 310 315 320
   Tyr Gln Ala Glu Leu Ser Gln Lys Pro Gly Glu Thr Leu Val Tyr Gly
   325 330 335
   Pro Phe Lys Lys Asp Leu Ile Val Lys Ile Asn Tyr Asn
   340 345
<210> 4
   <211> 20
   <212> DNA
   <213> artificial
<220>
   <223> primer
<400> 4
   atgcaatggc agtacccttc 20
<210> 5
   <211> 20
   <212> DNA
   <213> artificial
<220>
   <223> primer
<400> 5
   ctggtggcaa catcaaattg 20
<210> 6
<211> 21
   <212> DNA
   <213> artificial
<220>
   <223> primer
<400> 6
   attaccgtcg gttatatcgg c 21
<210> 7
   <211> 21
   <212> DNA
   <213> artificial
<220>
   <223> primer
<400> 7
   ataaacacaa tatggcgctc g 21
<210> 8
   <211> 32
   <212> DNA
   <213> artificial
<220>
   <223> primer
<400> 8
   cggatacgaa ttcatgatta cgctttttcg tt 32
<210> 9
   <211> 33
   <212> DNA
   <213> artificial
<220>
   <223> primer
<400> 9
   ttctcaaaag ctttgtcgtt cagttatagt tta 33
<210> 10
   <211> 48
   <212> DNA
   <213> artificial
<220>
   <223> primer
<400> 10
   cggatacgga tccatgttaa aaaaaacatt gttatctatg ttcgcaac 48
<210> 11
   <211> 43
   <212> DNA
   <213> artificial
<220>
   <223> primer
<400> 11
   ttctcaaaag ctttcagtta tagtttattt ttacgatgag atc 43
<210> 12
   <211> 20
   <212> DNA
   <213> artificial
<220>
   <223> primer
<400> 12
   agttaggctt tgtggcggac 20
<210> 13
   <211> 18
   <212> DNA
   <213> artificial
<220>
   <223> primer
<400> 13
   gttcaccgtc tatctcct 18
<210> 14
   <211> 46
   <212> DNA
   <213> artificial
<220>
   <223> primer
<400> 14
   cggtactctg aatgttaaaa aaaacattgt tatctatgtt cgcaac 46
<210> 15
   <211> 36
   <212> DNA
   <213> artificial
<220>
   <223> primer
<400> 15
   ttctcgatcc tcagtttagt ttatttttac gggatc 36
<210> 16
   <211> 62
   <212> DNA
   <213> artificial
<220>
   <223> primer
<400> 16
   aatccctctg ccaaagctct cctgctaaga aggggaaaat gtgtaggctg gagctgcttc 60
   ga 62
<210> 17
   <211> 60
   <212> DNA
   <213> artificial
<220>
   <223> primer
<400> 17
   taaatatgaa aatgccgggg tgttcccggc attttgctgt catatgaata tcctccttag 60
<210> 18
   <211> 16
   <212> DNA
   <213> artificial
<220>
   <223> primer
<400> 18
   gtaaaacgac ggccag 16
<210> 19
   <211> 17
   <212> DNA
   <213> artificial
<220>
   <223> primer
<400> 19
   caggaaacag ctatgac 17
<210> 20
   <211> 20
   <212> DNA
   <213> artificial
<220>
   <223> primer
<400> 20
   ttatacgcaa ggcgacaagg 20
<210> 21
   <211> 20
   <212> DNA
   <213> artificial
<220>
   <223> primer
<400> 21
   gatcttccgt cacaggtagg 20
<210> 22
   <211> 21
   <212> DNA
   <213> artificial
<220>
   <223> primer
<400> 22
   aatgtgtgga attgtgagcg g 21
<210> 23
   <211> 19
   <212> DNA
   <213> artificial
<220>
   <223> primer
<400> 23
   gccgacatga tccaactga 19

## Description

Research on avian pathogenic E. coli (APEC) has increased greatly over the years where the pathogen has been known to cause disease among chickens and other fowls which usually results in large economic losses for the poultry industry. APEC are mostly associated with infection of extraintestinal tissues in chickens, turkeys, ducks and other avian species. The most important disease syndrome associated with APEC begins as a respiratory tract infection and may be referred to as aerosacculitis or the air sac disease (Dho-Moulin M, Fairbrother JM (1999) Avian pathogenic Escherichia coli (APEC). Vet Res 30: 299-316.). This inevitably results in severe systemic infection leading to death of the animal infected. These pathogens have recently gained even more importance, now being classified under the category of extraintestinal pathogenic E. coli (ExPEC), a group which includes both human pathogens like the uropathogenic E. coli (UPEC) and newborn meningitic E. coli (NMEC) and animal pathogens, which in turn suggest the possibility of APEC having zoonotic potential (Ewers C, Li G, Wilking H, Kiessling S, Alt K, et al. (2007) Avian pathogenic,uropathogenic, and newborn meningitis-causing Escherichia coli: How closely related are they? Int J Med Microbiol 297: 163-176.).

Microbial pathogenicity is a complex phenomenon encompassing diverse mechanisms. There are, however, several common strategies that pathogenic organisms use to sustain themselves and overcome host barriers, one of them being the firm adhesion of the microorganism to host cells. Colonization is crucial to pathogenesis of bacteria, being the earliest stage during onset of the disease and the ability to adhere to host surfaces is by far the most vital step in the successful colonization by microbial pathogens. The presence of adhesins are said to be essential to the first steps of bacterial pathogenicity. A distinct family of adhesins called the adhesive pili or fimbriae, encoded by adhesin-gene clusters and assembled by the chaperone-usher pathway are said to be ubiquitous in Gram-negative organisms. Some of the well known fimbriae present among ExPEC strains are Type I fimbriae (fim), P fimbriae or the pilus associated with pyelonephritis (pap), curli fibre (csg), S fimbriae or the sialic acid-specific fimbriae (sfa), F1C fimbriae (foc), afimbrial adhesin/ Dr antigen-specific adhesin (afa/dra), heat-resistant agglutinin (hra) and others (see Ewers et al.). Each of these adhesins recognizes a specific receptor although as a group they share common genomic organization, assembly and even quaternary structural traits.

The role of some of these adhesins during APEC infection is well recognized. It has been reported that bacterial colonization of the respiratory tract is mediated by fimbrial adhesins, and studies have shown that type 1 and P fimbriae are expressed in vivo by bacteria colonizing the lung, air sacs and internal organs of chickens during infection. Bacterial adhesion to pharyngeal epithelial cells of chickens was also found to be inhibited by the presence of D-mannose, demonstrating the role of type 1 fimbriae during the early stages of the disease. No expression of P fimbriae was seen in the chicken trachea, suggesting that they may be important only in later stages of infection. In another study, it was seen that 99% of E. coli isolated from diseased birds possessed the csgA gene responsible for curli biosynthesis (see Dho-Moulin et al.).

Although there is increased knowledge about fimbrial adhesins and other colonization factors including the role they might play during infection, further investigation is still required to completely elucidate the function of each adhesin and its specific role in pathogenesis. In addition, the availability of complete genome sequences for some ExPEC pathogens like UPEC strain UTI89 (O18:K1:H7) and APEC strain APEC_O1 (O1:K1:H7) has drawn our attention to the large increase in the number of genes with unknown function, possibly involved in adhesion and colonization, and whose contribution to virulence on the whole is presently merely hypothetical (Johnson TJ, Kariyawasam S, Wannemuehler Y, Mangiamele P, Johnson SJ, et al. (2007) The genome sequence of avian pathogenic Escherichia coli strain O1:K1:H7 shares strong similarities with human extraintestinal pathogenic E. coli genomes. J Bacteriol 189: 3228-3236.). Determination of the function of these novel genes will lead to a deeper understanding of hostpathogen interactions during the initial stages of APEC infection.

Previously our laboratory applied Signature-tagged mutagenesis (STM) to APEC in a chicken systemic infection model which led to the successful identification of genes crucial to systemic infection in chickens, however no adhesins were identified (Li G, Laturnus C, Ewers C, Wieler LH (2005) Identification of genes required for avian Escherichia coli septicemia by signature-tagged mutagenesis. Infect Immun 73: 2818-2827.).

It is an aspect of the present invention to identify novel structures that play a role during infection of vertebrates with ExPEC and the use thereof in treatment and/or diagnosis of infectious diseases.

The present invention provides an isolated nucleic acid for use in treatment or diagnosis of infectious diseases, wherein the infections disease is associated with infection of a vertebrate with extraintestinal pathogenic APEC bacteria wherein the nucleic acid comprises or consists of:
- the nucleic acid sequence of the *yqi* gene with SEQ ID NO. 1 or the reverse complement thereof;
- a nucleic acid sequence with at least 95% sequence identity to SEQ ID NO. 1 or a reverse complement thereof;
- a nucleic acid sequence hybridizing under stringent conditions to the nucleic acid sequence with SEQ ID NO. 1 or to its reverse complement.

In a preferred embodiment, a nucleic acid is provided, wherein the nucleic acid comprises or consists of:
- the nucleic acid sequence of the *yqi* gene cluster with SEQ ID NO. 2 or the reverse complement thereof;
- a nucleic acid sequence with at least 95% sequence identity to SEQ ID NO. 2 or a reverse complement thereof; or
- a nucleic acid sequence hybridizing under stringent conditions to the nucleic acid sequence with SEQ ID NO. 2 or to its reverse complement.

The present invention is also directed to an isolated peptide for use in treatment or diagnosis of infectious diseases wherein the infections disease is associated with infection of a vertebrate with extraintestinal pathogenic APEC bacteria, wherein the infections disease is associated with infection of a vertibrate with extraintestinal pathogenic ADEC bacteria wherein the polypeptide comprises or consists of an amino acid sequence encoded by a nucleic acid of one of claims 1 to 3, preferably by the nucleic acid sequence of the *yqi gene* with SEQ ID NO. 1 or by a nucleic acid sequence with at least 95% sequence identity to SEQ ID NO. 1.

Surprisingly, a signature-tagged mutagenesis (STM) screen in APEC in a chicken systemic infection model led to the successful identification of the *yqi* adhesin gene as being crucial to systemic infection in chickens and as playing a crucial role in the initiation of such APEC infection. Deletion of the *yqi* adhesin gene in APEC results in a significant decrease in colonization of cells *in vitro* and of the chicken lung *in vivo.* Unexpectedly it was found that the prevalence of the *yqi* gene with the SEQ ID NO. 1 among a large collection of *E. coli* strains revealed a particularly high incidence of this gene in ExPEC strains like e.g. APEC, UPEC, NMEC and septicaemia associated *E- coli* (SePEC), whereas this gene was found only infrequently among non-pathogenic *E. coli* strains and was found to be completely absent among intestinal pathogenic *E. coli* strains.

Thus, it appears that a nucleic acid comprising the *yqi* adhesin gene and/or a polypeptides encoded by said gene and/or an antibody binding specifically to said polypeptide can be used in treatment and/or diagnosis of infectious diseases, in particular of an infectious disease associated with infection of a vertebrate with ExPEC bacteria, e.g. with bacteria of APEC, UPEC, SePEC and/or NMEC strains.

The nucleic acid of the invention is isolated. An "isolated" nucleic acid is one that is substantially separated from other nucleic acid molecules, which are present in the natural source of the nucleic acid (e.g.,sequences encoding other polypeptides). Preferably, an "isolated" nucleic acid is free of some or all of the sequences, which naturally flank the nucleic acid in its naturally occurring replicon, i.e. sequences located at the 5' and 3' ends of the nucleic acid of the invention. For example, a cloned nucleic acid is considered isolated. In various embodiments, the isolated nucleic acid of the invention can contain less than about 5 kb, 4 kb, 3 kb, 2kb, 1 kb, 0.5 kb, or 0.1 kb of nucleotide sequences which naturally flank the nucleic acid molecule in genomic DNA of the bacterial cell from which the nucleic acid is derived. A nucleic acid is also considered isolated if it has been altered by human intervention, or placed in a locus or location that is not its natural site, or if it is introduced into a cell e.g. by transfection or transformation. Moreover, an "isolated" nucleic acid, such as a cDNA molecule, can be free from some of the other cellular material with which it is naturally associated, or culture medium when produced by recombinant techniques, or chemical precursors or other chemicals when chemically synthesized. Specifically excluded from the definition of "isolated nucleic acid" are: naturally- occurring chromosomes (such as chromosome spreads), genomic libraries, and whole cell genomic DNA or whole cell RNA preparations of naturally occurring sources (including whole cell preparations that are mechanically sheared or enzymatically digested). Nucleic acids and/or polypeptides of the present invention may be provided in isolated form, i.e. purified from their natural environment, preferably in substantially pure or homogeneous form or free or substantially free of nucleic acid or genes of the species of origin other than the desired sequence.

Nucleic acid according to the present invention may include DNA, RNA, mixtures and/or functional substituents thereof, particularly cDNA, genomic DNA and mRNA and may be wholly or partially synthetic. The nucleic acids of the invention comprise single stranded or wholly or partially double stranded poly-nucleotide sequences. The term "isolated nucleic acid" encompasses all these possibilities. For the purpose of the present invention, where a DNA sequence is specified, e.g. with reference to a particular SEQ ID NO., unless the context requires otherwise, the RNA equivalent, with U substituted for T where it occurs, is encompassed. The nucleic acid of the invention may be produced by any means, including genomic preparations, cDNA preparations, in vitro synthesis, PCR, RT-PCR, and/or *in vitro* or *in vivo* transcription.

The isolated nucleic acid of the invention may comprise or consist of at least one nucleic acid sequence selected from:
- the nucleic acid sequence of the *yqi* adhesin gene with SEQ ID NO. 1 or the reverse complement thereof;
- a nucleic acid sequence with at least 95%, preferably at least 99%, sequence identity to SEQ ID NO. 1 or a reverse complement thereof, wherein sequence identity is determined over a sequence segment of the nucleic acid sequence with SEQ ID NO. 1 of at least 18 consecutive bases, preferably over a sequence segment of at least 20 consecutive bases, more preferably over a sequence segment of at least 50 consecutive bases, even more preferably over a sequence segment of at least 250 bases, most preferably over the whole sequence of SEQ ID NO. 1;
iii) a nucleic acid sequence hybridizing under stringent conditions to the nucleic acid sequence with SEQ ID NO. 1 or to its reverse complement.

Sequence identity between two nucleic acid sequences is understood as meaning the identity of the respective sequences over a given sequence length in each case, which is calculated by comparison with the aid of the GAP program algorithm (Wisconsin Package Version 10.0, University of Wisconsin, Genetics Computer Group (GCG), Madison, USA), setting the following parameters:
Gap Weight: 12
Length Weight: 4
10 Average Match: 2.912
Average Mismatch: -2.003

For example, a sequence which has at least 95% identity with the sequences of SEQ ID NO. 1 on nucleic acid basis is understood as meaning a sequence which, upon comparison with the sequence SEQ ID NO. 1 by the above program algorithm with the above set of parameters, has at least 95% identity.

The isolated nucleic acid of the invention may comprise or consist of at least one nucleic acid sequence selected from a nucleic acid sequence hybridizing under stringent conditions to the nucleic acid sequence with SEQ ID NO. 1 or to its reverse complement. The term "stringent hybridization conditions" is to be understood broadly and means stringent hybridization conditions as described e.g. by Sambrook J. et al. in Molecular Cloning-A Laboratory Manual, 2nd edition, Cold Spring Harbor Laboratory Press, 1989, pages 9.31-9.57 or in Current Protocols in Molecular Biology, John 10 Wiley & Sons, N.Y. (1989), 6.3.1-6.3.6.

For example, the conditions during the washing step(s) can be selected from the range of conditions limited by those of a lower stringency level (with approximately 2*SSC at 50 °C) and of a high stringency level (with approximately 0.2*SSC at 50 °C, preferably at 65 °C) (20*SSC: 0.3 M sodium citrate, 3 M NaCl, pH 7.0). In addition, the temperature during the washing step can be raised from low-stringency conditions at room temperature, approximately 22 °C, to more stringent conditions at approximately 65 °C. Both parameters, the salt concentration and the temperature, can be varied simultaneously, and it is also possible for one of the two parameters to be kept constant and only the other to be varied. It is also possible to employ denaturing agents such as, for example, formamide or SDS during the hybridization. Hybridization in the presence of 50% formamide is preferably carried out at 42 °C. Some exemplary conditions for hybridization and washing steps are given below:
(1) Hybridization conditions with for example
   a) 4*SSC at 65 °C, or
   b) 6*SSC, 0.5% SDS, 100 µg/ml denatured fragmented salmon sperm DNA at 65 °C, or
   c) 4*SSC, 50% formamide, at 42 °C, or
   d) 2* or 4*SSC at 50 °C, or
   e) 2* or 4*SSC, 30 to 40% formamide at 42 °C, or
   f) 6*SSC at 45 °C, or
   g) 0.05 M sodium phosphate buffer pH 7.0, 2 mM EDTA, 1% BSA and 7% SDS.
(2) Washing steps with for example
   a) 0.1*SSC at 65 °C., or
   b) 0.1*SSC, 0.5% SDS at 68 °C, or
   c) 0.1*SSC, 0.5% SDS, 50% formamide at 42 °C, or
   d) 0.2*SSC, 0.1% SDS at 42°C, or
   e) 2*SSC at 65 °C, or
   f) 40 mM sodium phosphate buffer pH 7.0, 1% SDS, 2 mM EDTA.

Conditions that are applied during a PCR reaction and that allow for specific annealing of a PCR primer to its target sequence are also encompassed by the term "specific hybridizing conditions".

In a preferred embodiment, the nucleic acid of the invention is a nucleic acid, wherein the nucleic acid comprises or consists of:
- the nucleic acid sequence of the *yqi* gene cluster with SEQ ID NO. 2 or the reverse complement thereof;
- a nucleic acid sequence with at least 95%, preferably at least 99%, sequence identity to SEQ ID NO. 2 or a reverse complement thereof; or
- a nucleic acid sequence hybridizing under stringent conditions to the nucleic acid sequence with SEQ ID NO. 2 or to its reverse complement.

The nucleic acid of the invention may comprise or consists of a nucleic acid sequence selected from SEQ ID NO. 1, 2, 4 to 15 or a reverse complement thereof.

In another aspect, the present invention is directed to an isolated peptide, wherein the polypeptide of the invention comprises or consists of an amino acid sequence encoded by a nucleic acid of the invention, preferably the polypeptide of the invention comprises or consists of an amino acid sequence encoded by the nucleic acid sequence with SEQ ID NO. 1 or by a nucleic acid sequence with at least 95%, preferably with at least 99%, sequence identity to SEQ ID NO. 1.

In a particular preferred embodiment the polypeptide of the invention comprises or consists of the amino acid sequence with SEQ ID NO. 3.

The term "polypeptide" as used herein is an art-recognized term which refers to a chemical structure comprising or consisting of a polymer of amino acids, wherein at least a fraction of the amino acids are linked via peptide bonds. The term "polypeptide" as used herein refers to peptides with an amino acid sequence of at least 8 amino acids and also encompasses complete proteins. The polypeptide of the invention may consist solely of proteinergic amino acids, however, the polypeptide of the invention also encompasses polypeptides comprising or consisting of amino acids that are not naturally ocuring in proteins.

The present invention is also directed to an antibody specific for a polypeptide of the invention, preferably specific for a polypeptide comprising or consisting of an amino acid sequence of SEQ ID NO. 3. For the purpose of the present invention, the term "antibody" is not limited to an antibody with the classical heavy and light chain architecture. The terms "antibody" or "antibodies" as used herein are art-recognized terms and are understood to refer to molecules or active fragments of molecules that specifically bind to given antigens, i.e. to a polypeptide of the invention.

As used herein the terms "specifically binds" or "specific for" in reference to an antibody, a nucleic acid, an aptamer or any other class of specific binder means that the molecule binds to its target with greater affinity than it does to a structurally different epitope.

"Antibodies" are intended within the scope of the present invention to include monoclonal antibodies, polyclonal antibodies, chimeric, single chain, bispecific, human and humanized antibodies as well as active fragments thereof. Examples of active fragments of antibodies include separated light and heavy chains, Fab, Fab/c, Fv, scFv, diabodies, Fab' and F(ab')2 fragments, including epitope-binding fragments of any of the antibodies or fragments mentioned above.

The nucleic acid, the polypeptide and/or the antibody of the invention are suitable for use in treatment or diagnosis of infectious diseases. Such infectious diseases comprise diseases which are associated or caused by infection of a vertebrate with extraintestinal pathogenic *E. coli* bacteria (ExPEC), in particular with bacteria of APEC, UPEC, SePEC and/or NMEC strains. Preferably avian and/or mammalian vertebrates are treated and/or diagnosed.

The nucleic acid, the polypeptide and/or the antibody of the invention can be used in treatment of such infectious diseases. As used herein, the term "treating" refers to reversing, alleviating or inhibiting the progress of a disease, disorder or condition, or one or more symptoms of such disease, disorder or condition, to which such term applies. As used herein, "treating" may also refer to decreasing the probability or incidence of the occurrence of a disease, disorder or condition in a mammal as compared to an untreated control population, or as compared to the same mammal prior to treatment. For example, as used herein, "treating" may refer to preventing a disease, disorder or condition, and may include delaying or preventing the onset of a disease, disorder or condition, or delaying or preventing the symptoms associated with a disease, disorder or condition. As used herein, "treating" may also refer to reducing the severity of a disease, disorder or condition or symptoms associated with such disease, disorder or condition prior to a mammal's affliction with the disease, disorder or condition. Such prevention or reduction of the severity of a disease, disorder or condition prior to affliction relates to the administration of the composition of the present invention, as described herein, to a subject that is not at the time of administration afflicted with the disease, disorder or condition. As used herein "treating" may also refer to preventing the recurrence of a disease, disorder or condition or of one or more symptoms associated with such disease, disorder or condition. The terms "treatment" and "therapeutically," as used herein, refer to the act of treating, as "treating" is defined above.

The antibodies of the invention may be used to block binding of the *yqi* adhesin protein to its receptor and thereby may affect and reduce the rate of infection with ExPEC bacteria. The antibodies may be conjugated to an effector molecule allowing to kill or deactivate bacteria expressing the *yqi* adhesin protein.

The polypeptide of the invention may be used as targeting structure to allow efficient and specific targeting of the ExPEC bacteria in order to detect, deactivate or kill these bacteria. The polypeptide of the invention may also be used to treat infectious diseases by its use in the manufacture of a vaccine against infection with ExPEC bacteria. In particular the nucleic acid of the invention or the polypeptide of the invention can be used in the manufacture of a vaccine for use in treatment, e.g. therapy or prophylaxis, of a vertebrate against infection with ExPEC bacteria, e.g. with bacteria of APEC, UPEC, SePEC and/or NMEC strains.

The nucleic acid of the invention may be used as a target in order to site-direct a therapeutic molecule to an area where infection takes place or has already occured. Alternatively the nucleic acid of the invention as such may be used directly in such treatment, e.g. as antisense molecule, RNAi compound or as targeting modality of a conjugate comprising an effector domain that upon specific binding is able to excert a therapeutic effect.

The nucleic acid, the polypeptide and/or the antibody of the invention can be used in diagnosis of an infectious disease. Preferably the infectious disease is a disease associated and/or caused by infection of a vertebrate with ExPEC bacteria, e.g. with bacteria of APEC, UPEC, SePEC and/or NMEC strains.

The acid, the polypeptide and/or the antibody of the invention can be used in differential diagnosis of infection of a vertebrate by allowing discrimination between infection with ExPEC bacteria, e.g. infection with APEC, UPEC, SePEC and/or NMEC, versus infection with intestinal pathogenic E. coli, like e.g. EPEC or EMEC. Since intestinal pathogenic *E. coli* bacteria lack the *yqi* gene, it can easily be distinguished between an infection with ExPEC and an infection with intestinal pathogenic *E. coli* simply by checking for the presence of a nucleic acid of the invention or a polypeptide of the invention.

The diagnosis or differential diagnosis may comprise the following steps:
- provision of a probe: The term "probe" refers to a composition that is to be investigated for the presence of the nucleic acid of the invention or the polypeptide of the invention and which, in case of an infection, may contain infected or infectious material. Preferably, the probe encompasses biological and/or medical material, i.e. material derived from a biological source like e.g. an organism or cell or a component of an organism (e.g. tissue) or a cell. The material may be subjected to additional treatment steps prior to use of said material as probe in the method of diagnosis, e.g. in order place the material in a condition which allows detection of the nucleic acid or the polypeptide of the invention. Preferably the probe comprises or consists of material derived from a tissue or body fluid. Said body fluid may be blood, plasma, serum, urine, feces, synovial fluid, interstitial fluid, lymph, saliva, sudor, spinal fluid and/or lacrimal fluid. Preferably said probe or body fluid is of vertebrate origin, more preferably of avian or mammalian origin, e.g. human origin.
- contacting the probe with a binder, wherein the binder is specific for a nucleic acid of the invention or a polypeptide of the invention: Said contacting is performed under conditions which allow for specific binding of the binder to said nucleic acid and/or polypeptide of the invention.
- detection of the binder being specifically bound to the nucleic acid of the invention and/or the polypeptide of the invention: Upon specific binding of the binder to the nucleic acid or polypeptide of the invention, the presence of such conjugates is detected. The choice of binders and corresponding detection means is coordinated such that proper detection of specific binding events is secured. The binder may be a nucleic acid hybridizing under stringent conditions to a nucleic acid of the invention, like e.g. a PCR primer or a hybridization probe for a northern or southern hybridization. The binder specifically binding to a nucleic acid or a polypeptide of the invention may be an aptamer or an antibody, in particular the binder may be an antibody of the invention. Depending on the choice of binder the method of detection of specific binding is laid out accordingly. Steps of the method of diagnosis may be laid out as PCR or hybridization method and the binder may be a nucleic acid being complementary or hybridizing under stringent conditions to a nucleic acid of the invention. Alternatively steps of the method of diagnosis may be laid out as ELISA, RIA, western blot, strip assay or the like and the binder may be an aptamer or an antibody, e.g. an antibody of the invention.

Alternatively, the nucleic acid or the polypeptide of the invention may be contacted with a probe and it is detected whether said probe comprises a binder binding specifically to said nucleic acid or polypeptide of the invention. Such a binder may be an antibody specific for the polypeptide of the invention comprised in the probe. In case a vertebrate is infected or was infected with ExPEC bacteria , said vertebrate may have developped antibodies against strucutres of said ExPEC bacteria like e.g. the *yqi* adhesin protein. By use of the nucleic acid or polypeptide of the invention, it is possible to detect such antibodies in a probe of such a vertebrate and thereby to diagnose an infection, to determine an antibody titer and e.g. to check for successful immunization with a vaccine based on the *yqi* adhesin protein.

The present invention is also directed to a kit for use in diagnosis of an infectious disease, preferably a disease associated with infection of a vertebrate with extraintestinal pathogenic *E. coli* (ExPEC) bacteria, e.g. with bacteria of APEC, UPEC, SePEC and/or NMEC strains, wherein the kit comprises a nucleic acid or polypeptide of the present invnetion, a nucleic acid being complementary to a nucleic acid of the invention, or an antibody of the invention, and optionally a manual and/or consumales configured for use in such diagnosis. The kit of the invention may comprise a nucleic acid or polypeptide of the present invnetion, a nucleic acid being complementary to a nucleic acid of the invention, or an antibody of the invention immobilized to a solid support.

According to another aspect, the present invention is directed to an isolated nucleic acid consisting of a nucleic acid sequence with SEQ ID NO. 1 or SEQ ID NO. 2. The present invention is also directed to an isolated polypeptide consisting of an amino acid sequence with SEQ ID NO. 3.

In the follwoing the present invention will be explained in more detail by way of examples.

### FIGURES:

- **Figure 1.**: Graphic illustrating growth of transposon mutant EA7F9 in competition with IMT5155 and separately in Luria Bertani (LB) growth medium. A competition index (Cl) *in vitro* was calculated at a time point of 4 h.
- **Figure 2.**: Physical map showing genomic organization of the *yqi* adhesin gene cluster in APEC strain IMT5155. A hypothetical protein preceeds the putative outer membrane usher protein, followed by the putative chaperone and finally the putative adhesin.
- **Figure 3.**: Dendrogram showing distances between *yqi* gene sequences among strains belonging to different sequence types (ST: Sequence type; STC: Sequence type complex)
- **Figure 4A.**: Bacterial adhesion to chicken fibroblast cells 1.5 h and 3 h after infection with an MOI=100. Differences between IMT5155 and IMT5155Δ*yqi* were statistically significant with a *p*<0.005 at 1.5h and *p*<0.05 at 3h.
- **Figure 4B.**: Bacterial adhesion to polarized Madin Darby canine kidney (MDCK-1) cells 3 h after infection with an MOI=100. The difference between IMT5155Δ*yqi* and IMT5155Δ*yqi* (pDSK602:*yqi*) was significant with a *p*<0.04.
- **Figure 5A.**: Bacterial colonization of the chicken lungs 24 h after intra-tracheal infection with 10⁶ CFU of bacteria. Differences between IMT5155 and IMT5155Δ*yqi* were statistically significant with a *p*<0.05 (n=6). Strain IMT11327 is the negative control.
- **Figure 5B.**: Bacterial colonization of the chicken lungs 24 h after intra-tracheal infection with 10⁹ CFU of bacteria. Differences between IMT5155 and IMT5155Δ*yqi* were statistically significant with a *p*<0.02 (n=6). Strain IMT11327 is the negative control.
- **Figure 6.**: Bacterial re-isolation of IMT5155, IMT5155Δ*yqi* and IMT11327 from the lungs, spleen, liver, heart, kidneys and brain 24 h after intra-tracheal infection with 10⁹ CFU of bacteria (n=6). Absence of columns indicates that no bacteria were isolated from the organ.
- **Figure 7.**: Electron micrographs showing negatively stained afimbriate strain *E. coli* AAEC189 (pKESK:*yqi*_4975_XB) over-expressed with the *yqi* adhesin gene cluster at a magnification of 45,000x (A), 65,000x (B) and 100,000x (C), negative control afimbriate strain *E. coli* AAEC189 (D), wild type fimbriated *E. coli* strain IMT5155 (E). The arrows indicate the location of the fimbriae.

### EXAMPLES:

### Materials and methods

### Bacterial strains, plasmids and growth conditions

All *E. coli* strains and plasmids used are listed in table 1. The wild type APEC isolate IMT5155 (02:K1:H5) was obtained from internal organs of a laying hen clinically diagnosed with systemic APEC infection during an outbreak in the northern part of Germany in the year 2000 (Li G, Laturnus C, Ewers C, Wieler LH (2005) Identification of genes required for avian Escherichia coli septicemia by signature-tagged mutagenesis. Infect Immun 73: 2818-2827.). The strain possesses a number of virulence-associated genes typical of ExPEC strains, curli fibre gene (crl), type 1 fimbriae *(fimC),* temperature-sensitive haemagglutinin *(tsh),* heme receptor gene *(chuA),* outer membrane protein *(ompA),* invasion of brain endothelium *(ibeA),* genetic island associated with newborn meningitis *(gimB),* colicin V plasmid (colV) and is classified into the B2 phylogenetic group, and multi locus sequence type 140 of the ST95 complex. An additional *E. coli* strain IMT11327 (Ont:H16), isolated from the intestine of a clinically healthy chicken, was used as a non-virulent control in both *in vivo* and *in vitro* assays, while *E. coli -* K12 strain (MG1655) was used as a negative control *in vitro.* IMT5155 was used for all genetic manipulation studies. All isolates used in the prevalence studies are part of a strain collection in our laboratory obtained from various sources (Ewers C, Li G, Wilking H, Kiessling S, Alt K, et al. (2007) Avian pathogenic, uropathogenic, and newborn meningitis-causing Escherichia coli: How closely related are they? Int J Med Microbiol 297: 163-176.).
Bacterial strains were routinely cultured at 37°C in Luria-Bertani (LB) broth and on LB agar plates with appropriate antibiotics when required in the following concentrations: Kanamycin (Kan), 50µg/ml; Nalidixin (Nal), 30µg/ml; Ampicillin (Amp), 50µg/ml; Chloramphenicol (Cm), 30µg/ml; Spectinomycin (Spec) 50µg/ml. All strains were stored at -70°C in LB broth with 10% (v/v) glycerol until further use.

### PCR analyses

All oligonucleotide primers are listed in table 2. Unless otherwise specified, *E. coli* isolates, from which DNA was to be used as the template for PCR amplification, were grown overnight in Luria-Bertani broth at 37°C and DNA was released from whole organisms by boiling for 10 minutes. After centrifugation, 2 µl of the supernatant was taken as template DNA and added to a 25 µl reaction mixture containing 0.1 µl of each primer pair in a 10 pmol concentration, 0.3 µl 10mM of the four deoxynucleoside triphosphates (Sigma-Aldrich Chemie GmbH, Munich Germany), 2.5 µl of 10x PCR buffer, 1 µl of 50mM magnesium chloride and 1 unit of Taq-Polymerase (Rapidozym GmbH, Berlin Germany). The samples were subjected to 25 cycles of amplification in a thermal cycler (GeneAmp PCR system, Applied Biosystems, Darmstadt, Germany). The amplification products were analyzed by gel electrophoresis on a 1.5% agarose gel (Biodeal, Markkleeberg, Germany), stained with ethidium bromide and photographed on exposure to UV.

### Establishment of a lung colonization model of infection

A modified lung colonization model of infection was established based on the existing systemic infection model, for the purpose of screening STM mutant pools for adhesion and colonization factors as described previously (Antao EM, Glodde S, Li G, Sharifi R, Homeier T, et al. (2008) The chicken as a natural model for extraintestinal infections caused by avian pathogenic Escherichia coli (APEC). Microb Pathog 45: 361-369.). The lung infection model differed from the systemic infection model in that, the infection dose was lowered to 10⁶ CFU such that no systemic infection was induced.
Briefly, five-week old white leghorn specific pathogen free (SPF) chickens (Lohmann Tierzucht GmbH, Cuxhaven, Germany) were used for infection purposes. Groups of 10 chickens were infected intra-tracheally with a 0.5 ml suspension containing 10⁶, 10⁵, 10⁴ and 10³ CFU of the virulent strain IMT5155 respectively. IMT11327 was used as a negative control during infection. Chickens were euthanized 24h post infection, and a clinical score was determined which monitored the infection ranging from score 0 (no symptoms) to score 4 (severe symptoms). An organ lesion score was likewise determined with a minimum and maximum score of 1 and 5 in the lungs respectively depicting the severity of lesions in the lungs, and a score of 0 (no hyperplasia) or 1 (hyperplasia) in the spleen. Bacteria were isolated from the lungs and spleen as follows: Organ samples were weighed, suspended in phosphate-buffered saline (1ml/g) and homogenized with an Ultra-Turrax apparatus. Serial dilutions were plated out onto LB agar plates which were then incubated at 37°C for 24h. Colonies were then counted to determine the CFU per gram in each organ. An infection dose of 10⁶ was made the infection dose of choice in subsequent infections, since the number of re-isolated bacteria with this infection dose was optimal for STM analyses (data not shown).

### Transposon Tn5km2 mutagenesis and identification of a novel adhesin

Insertion mutagenesis of APEC strain IMT5155 was performed randomly by using transposon pUTmini-Tn5km2 as previously described (Li G, Laturnus C, Ewers C, Wieler LH (2005) Identification of genes required for avian Escherichia coli septicemia by signature-tagged mutagenesis. Infect Immun 73: 2818-2827.). A transposon mutant library of strain IMT5155 was generated in this study and screened as described previously by Li et al, in the chicken lung colonization model of infection in the search for colonization and adhesion factors crucial to APEC infection. A mutant EA7F9, was selected in this screen and the disrupted gene *yqi,* encoding a putative adhesin, was identified using arbitrary PCR also described previously (Li et al.). This adhesin, temporarily renamed ExPEC adhesin I (EA/I) was further characterized for its role in APEC pathogenesis.

### Functional characterization of ExPEC adhesin I(EA/I)

### In vitro and in vivo competition assays

*In vitro* and *in vivo* competition assays were performed by mixing cultures of mutant and wild type strains with an OD₆₀₀=1 in a ratio of 1:1. For *in vitro* assays, the bacterial mixture was incubated in LB broth for 4h at 37°C and then plated onto LB plates with and without Kanamycin and Nalidixin. For *in vivo* assays, four chickens were infected with 10⁶ CFU/ml of this bacterial mixture. At 24h post infection chickens were euthanized, lungs homogenized and serial dilutions plated onto LB plates with and without Kanamycin and Nalidixin for selection of mutant and wild type strain respectively. A competitive index (CI) was calculated by dividing the output ratio (CFU mutant: CFU wild type) by the input ratio (CFU mutant: CFU wild type) at 0h and 4h or 24h for *in vitro* and *in vivo* assays respectively.

### Sequencing of a 4975bp EA/1 gene cluster region in IMT5155

In order to sequence the EA/I gene cluster (*yqi*) in IMT5155, the 4975bp region was amplified using primers IMT3045 and IMT3046 and the PCR product was cloned into pCR2.1 TOPO vector according to the standard TOPO cloning manual (Invitrogen GmbH, Karlsruhe, Germany). The cloned product was transformed into *E. coli* TOP10 by electroporation and plated out on LB with Kanamycin to select for positive clones. Colonies were tested for the presence of the *yqi gene* cluster (4975bp) using standard primers IMT1560 and IMT1561. The plasmid containing the insert was isolated from the host strain and sequenced commercially by LGC's AGOWA Genomics, Berlin, Germany.

### Generation of an isogenic yqi mutant

To generate a knock-out mutant of the fimbrial adhesin in IMT5155, the *yqi* gene was replaced with a Chloramphenicol resistance cassette using the lambda red recombinase system. The Chloramphenicol acetyl transferase (CAT) gene was amplified from plasmid pKD3 using PCR with primers IMT2510 and IMT2511 (Table 2), part of which have sequence homology to the *yqi* flanking region. The PCR product was purified on a 1 % agarose gel and 2 µl of the sample was transformed by electroporation into IMT5155 containing the lambda red recombinase expression plasmid pKD46. After electroporation, samples were incubated at 28°C for 1h in SOC broth and plated on LB agar with Chloramphenicol to select for positive clones (CAT). After overnight incubation at 37°C, transformants were selected and tested for loss of the *yqi* gene using PCR with primer pairs IMT718/IMT2558 and IMT719/IMT2559 (Table 2).

### Complementation of yqi

For complementation and over-expression studies the IMT5155 *yqi* gene (1050bp) PCR product was cloned into pCR2.1 TOPO vector according to the standard TOPO cloning manual (Invitrogen GmbH, Karlsruhe, Germany) using primers IMT2910 and IMT2911 (Table 2) with restriction enzyme recognition sites *EcoRI* and *HindIII,* and transformed into *E. coli* TOP10 via electroporation. Positive colonies were selected on LB agar with Kanamycin and tested for the presence of *yqi* using standard primers IMT1560 and IMT1561 (Table 2). The TOPO vector with the IMT5155 *yqi* insert was commercially sequenced by LGC's AGOWA Genomics, Berlin, Germany to determine that the sequence was in frame. The pCR2.1 TOPO:*yqi* vector and expression vector pDSK602 were then digested with restriction enzymes *EcoRI* and *HindIII* for 1 h at 37°C, and ligated using T4 DNA ligase overnight at 4°C. Three microliters of the ligation mix were then electroporated into *E. coli* TOP10 and plated out on LB agar containing spectinomycin. Colonies were tested for the presence of *yqi* using PCR with primers IMT2910 and IMT2911 (Table 2). The modified plasmid pDSK602 with the *yqi* insert was isolated from *E. coli* TOP10 and transformed into IMT5155Δ*yqi* to complement the gene deleted. In adhesion and colonization assays, the complemented strain was induced with IPTG in a final concentration of 100mM for expression of the protein.

### Adhesion assays in vitro with Chicken Fibroblast (CEC) and polarized Madin Darby Canine Kidney (MDCK-1) epithelial cell lines

Chicken fibroblast (CEC) cells were used between passages 6 and 9 and were seeded into 12-well microtitre plates at a density of ∼2 x 10⁵ cells per well and incubated at 37°C in an atmosphere containing 5% CO₂ without antibiotics prior to adhesion assays. Minimal essential cell culture medium (Pan^{™} Biotech GmbH, Aidenbach, Germany) with 5% Foetal calf serum (FCS) (Pan^{™} Biotech GmbH, Aidenbach, Germany) was used to grow cells. Monolayers were used after 6 days incubation.
Madin Darby Canine Kidney (MDCK-1) cells were used between passages 1 and 5. Cells were grown in Dulbecco's modified eagle's medium (DMEM) (Pan^{™} Biotech GmbH, Aidenbach, Germany) with 10% FCS and were incubated at 37°C in 5% CO₂. Transwell filter units (Costar) (Sigma-Aldrich Chemie GmbH, Munich Germany) contained a 1.12cm² porous filter membrane (0.4-µm pores) that had been treated for tissue culture. Filter units were incubated in 12-well microtitre plates (Costar) and were placed in DMEM containing 10% FCS for 1 h, at 37°C before seeding. 40 µl of a trypsinized MDCK cell suspension were added to each Transwell unit. Monolayers were used after 4 days incubation at which time there were around ∼3 x 10⁵ MDCK cells per filter.
For adhesion assays, bacteria were added to the appropriate wells in triplicate in medium without FCS at an MOI of 100, that is, -2-3 x 10⁷ bacteria per well. Microtitre plates were centrifuged at 250 x g for 10 minutes, and then incubated for 1.5h and 3h for CEC cells, and 3h for MDCK cells, after which the supernatant was discarded; cells were washed thrice with Phosphate buffered saline (PBS) and then plated out on LB agar to determine the number of adherent bacteria in each well.

### Animal infection studies

Animal experiments were also carried out to determine the colonization ability of strain IMT5155Δ*yqi* in comparison to IMT5155. IMT11327 was used as the negative control. Briefly, in an assay involving the lung colonization chicken model, groups of 6 five-week old chickens each were infected intratracheally with a bacterial suspension containing 10⁶ bacteria per ml. At 24h post infection, chickens were euthanized and dissected. A clinical and organ score was recorded and the lungs and spleen were homogenized.
In the chicken systemic infection model, groups of 6 five-week old chickens were infected intratracheally with a bacterial suspension containing 10⁹ bacteria per ml. At 24h post infection chickens were euthanized, dissected and a clinical and organ score was recorded. The lungs, heart, liver, kidneys, spleen and brain were homogenized.
All homogenates were appropriately diluted and plated out on LB agar and LB agar with antibiotics where required, to determine the number of bacteria colonizing the chicken lung and number of bacteria in internal organs during systemic infection.

### Over-expression of the 4975bp EA/I adhesin gene cluster in fimbrial negative E. coli strain AAEC189

The 4975bp *EA*/*1* adhesin gene cluster was amplified and the PCR product was cloned into pCR2.1 vector according to the standard TOPO cloning manual (Invitrogen GmbH, Karlsruhe, Germany) using primers IMT3259 and IMT3260 with restriction enzyme cutting sites *XbaI* and *BamHI,* and transformed into electro-competent *E. coli* TOP10 cells via electroporation. Positive clones were selected on LB agar with Kanamycin and tested for the presence of the 4975bp gene cluster using standard primers IMT1560 and IMT1561.
The plasmid pCR2.1-TOPO:*yqi*_4975_XB and expression vector pKESK-22 were digested with restriction enzymes *XbaI* and *BamHI,* and ligated using T4 DNA ligase overnight at 4°C and finally electroporated into electro-competent E.coli TOP10 cells and plated out on LB agar containing spectinomycin. Colonies were tested for the presence of the 4975 bp adhesin gene cluster using PCR with primers IMT3138 and IMT3139.
Plasmid pKESK*yqi*_4975_XB was isolated from *E. coli TOP10* and transformed into electro-competent *E. coli* AAEC189 (afimbriate *E. coli* strain) cells, to over-express the adhesin gene cluster.

### Electron Microscopy

*E. coli* strains AAEC189, AAEC189 (pKESK:*yqi*_4975_XB) and IMT5155 were grown in 5 ml Brain Heart infusion broth after inoculating with 150 µl of the respective overnight cultures. Strains AAEC189 and IMT5155 were used as negative and positive controls respectively. Strain AAEC189 (pKESK:*yqi*_4975_XB) was additionally given Kanamycin in its growth medium, and induced with 0.1M IPTG after 30 minutes of growth. All three strains were grown to an OD₆₀₀ of 2.5, allowing an induction time of 2.5h for strain AAEC189 (pKESK:*yqi*_4975_XB). One millilitre of bacterial culture was centrifuged at 8000 x g and the bacterial pellet was washed thrice with 1x PBS and then resuspended in 500 µl 1x PBS. Twenty microlitres of bacterial suspension were applied to Formvar-coated 200- mesh copper grids and stained with 1 % Uranyl acetate for 2 mins. Negatively stained preparations were examined on a Zeiss EM900 microscope.

### Prevalence of the EA/1 gene yqi among ExPEC, Intestinal pathogenic E. coli (IPEC) and commensals (A_{faecal} strains)

To determine the prevalence of *yqi* among *E. coli* strains, 406 avian pathogenic *E. coli* (APEC) strains, 138 uropathogenic *E. coli* (UPEC), 25 Newborn meningitic *E. coli* (NMEC), 19 Septicaemia associated *E. coli* (SePEC), 153 intestinal pathogenic *E. coli* (IPEC) and 159 faecal strains from clinically healthy chickens (A_{fecal}) were tested for the presence of the *yqi* gene using standard PCR reactions with primers IMT2512 and IMT2513 by way of amplification of a 400 bp region of the adhesin gene as described under PCR analyses. IMT5155 was used as a positive control, and IMT11327 as a negative control for all PCR reactions. Results were observed as a single clear band on an agarose gel and recorded as positive and negative for all strains respectively.

### Sequencing of the EA/1 gene yqi and evolutionary analysis

A total of 77 strains representing multi locus sequence types (MLST) ST12, ST73, ST95, ST104, ST135, ST140, ST141, ST358, ST363, ST368, ST372, ST390, ST416, ST417 and ST421 were selected for sequencing of the *yqi* gene (1050bp), from a previous study at the Institute of Microbiology and Epizootics involving MLST analysis, using primer pairs IMT3706/IMT3707 and IMT2512/IMT2559. The PCR products were sequenced commercially by LGC's AGOWA Genomics, Berlin, such that a double stranded sequence was obtained for each strain. Sequences were analysed using Kodon software available from Applied Maths. A phylogenetic tree showing distances between strains of different STs was calculated by a maximum parsimony algorithm using Kodon Software from Applied Maths.
The adhesin gene sequence of the strains APEC_O1 (APEC) (Accession: CP000468), CFT073 (UPEC) (Accession: AE014075), UTI89 (UPEC) (CP000243) was obtained from the available nucleotide database, and compared with sequenced strains in this study. Rates of non-synonymous (Dn) and synonymous (Ds) mutation were calculated using DnaSP 4.50.3 software for the sequenced adhesin gene among strains belonging to the ST95 complex in order to determine the Dn/Ds ratio for each locus as described previously.

### Statistical analysis

All statistical analysis for *in vivo* animal experiments and *in vitro* cell culture experiments were carried out using the software SPSS (Statistical package for the social sciences), version 15.0 by carrying out the non-parametric Mann-Whitney U-Test and the students t-test at the 95 % significance level (*p*<0.05).

### Results

### Attenuation of IMT5155 with mutation in ExPEC adhesin I(EA/I) gene yqi

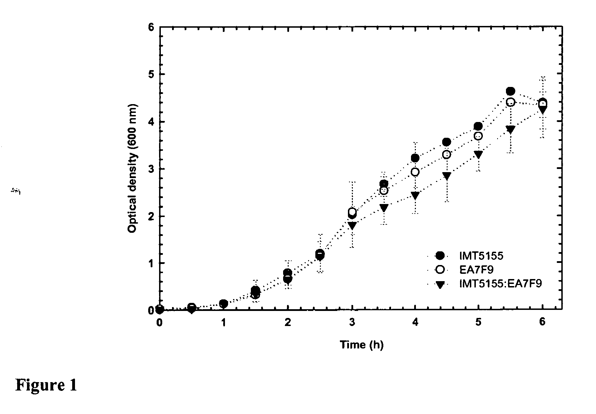

Among the many genes identified during the STM screen, was the ExPEC adhesin I (EA/I) gene, also annotated as *yqi. In vitro* competition assays of transposon-mutant EA7F9 versus wild type strain IMT5155 showed that there was no significant growth defect *in vitro* as determined by growth curves (Figure 1). An *in vitro* competition index of 1.2 was observed for EA7F9. On the other hand, the mutant was found to be attenuated in the chicken with an *in* vivo competition index of 0.6. This result confirmed the attenuation of mutant EA7F9 during STM screening.

### Sequencing of the EA/I gene cluster region in IMT5155

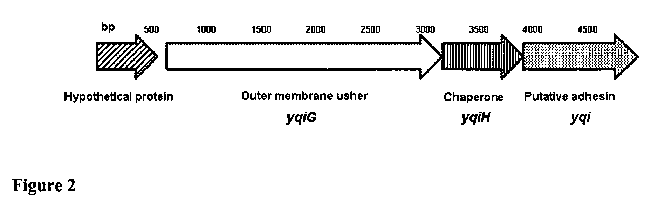

The putative *yqi* adhesin gene cluster was completely sequenced in APEC strain IMT5155. Genomic organization of the IMT5155 *yqi* adhesin gene cluster was as follows: the putative outer membrane usher preceded the periplasmic chaperone, followed by the adhesin gene. A conserved hypothetical protein gene precedes the usher gene which may code for the adhesin subunit protein although this has not yet been confirmed (Figure 2). This 4,975 bp region showed a hundred percent sequence identity with the UPEC and APEC *yqi* adhesin gene cluster in sequenced strains UTI89 (Accession: CP000243) and APEC_O1 (Accession: CP000468). Comparing the sequence with genomes available in the public database, this adhesin gene cluster is only found among ExPEC strains including APEC, UPEC and NMEC and is not harboured by intestinal pathogenic *E. coli* like enteropathogenic *E. coli* (EPEC) or enterohaemorrhagic *E. coli* (EHEC) or non pathogenic *E. coli.*

### EA/I plays a role in the adhesion of APEC to chicken fibroblasts and kidney epithelial cells in vitro

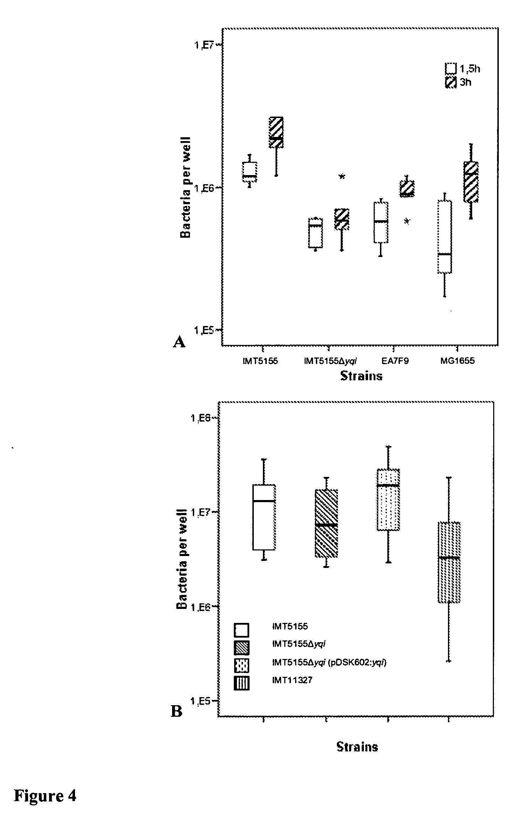

In order to determine the effect of EA/I in APEC, adhesion assays were initially performed *in vitro* using chicken fibroblast cells. Strain EA7F9 with a disruption in the *yqi* gene via a transposon (STM generated mutant) (Table 1), and strain IMT5155Δ*yqi* devoid of the ExPEC adhesin I gene were tested against the wild type pathogen IMT5155 and a negative control strain MG1655. Bacterial determination at two different time points revealed a reduction in the ability of both EA7F9 and IMT5155Δ*yqi* to adhere to fibroblast cells up to about forty percent of the total adhesion by IMT5155 (Figure 4A). MG1655 as expected also showed decreased adhesion ability when compared with IMT5155. An average CFU was calculated from three independent wells, and results were reproducible between adhesion experiments. Statistical analysis of the CFU values showed a significant difference between wild type IMT5155 and mutants tested, with a *p*<0.005 and *p*<0.05 at 1.5h and 3h respectively (Figure 4A).
To further confirm the role of *yqi* in APEC *in vitro,* adhesion assays were carried out using polarized Madin-Darby Canine-Kidney (MDCK-1) epithelial cells. Strains IMT5155Δ*yqi* and IMT5155Δ*yqi*+, a strain carrying a modified expression plasmid with the *yqi gene,* that is, the complemented mutant, were tested against IMT5155, the positive control, and IMT11327, a negative control strain of avian origin lacking the gene *yqi.* A reduction in colonization by IMT5155Δ*yqi* was seen, up to twenty percent of the total adhesion by IMT5155, 3h after infection (Figure 4B). The complemented strain IMT5155Δ*yqi* (pDSK602:*yqi*) regained its ability to adhere to MDCK-1 cells by introduction of the *yqi* gene when compared with its deletion mutant IMT5155Δyqi with a significance of *p*<0.04. All results were reproducible in consecutive adhesion experiments.

### Effect of EAlI in colonization of the chicken lung in vivo

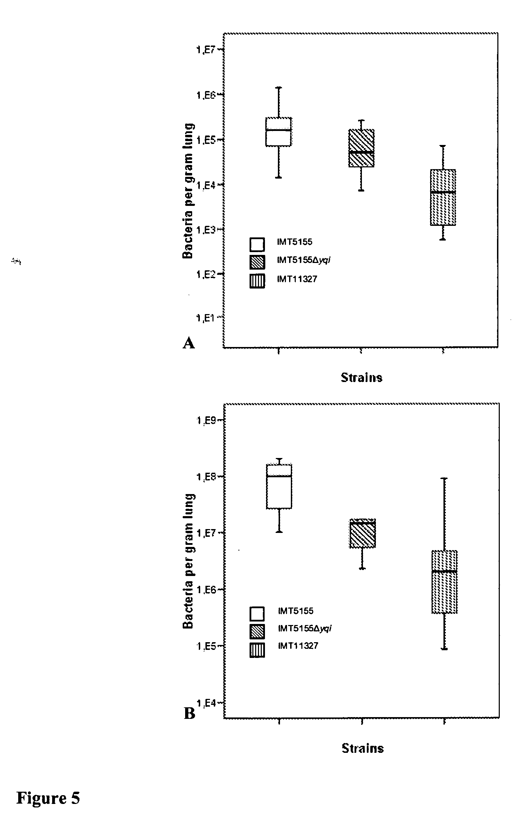

Colonization of the chicken lung *in vivo* was studied by infecting 5-week old chickens intratracheally, and isolating bacteria from the lungs 24h after infection. For this purpose, two different infection set ups were made use of, including the lung colonization model of infection and the systemic infection model. IMT5155 and IMT11327 served as positive and negative controls respectively. The results of the *in vivo* experiments confirmed the observations made *in vitro* in cell culture models described above. When chickens were infected with a dose of 10⁶ CFU of IMT5155Δ*yqi* in a model used to study colonization abilities of the chicken lung by various strains, a distinct reduction in re-isolated bacterial numbers was observed as compared to IMT5155 as depicted in figure 5A. Differences between strains were statistically significant with a *p*<0.05. The average lung score in chickens infected with IMT5155Δ*yqi* was 1.63 in comparison to 1.71 and 1.21 in chickens infected with IMT5155 and IMT11327 respectively. All chickens infected did not exhibit any clinical symptoms.
Moreover, when chickens were infected with a higher dose of bacteria, that is, 10⁹ CFU, in a model designed to induce systemic infection, there still was a significant difference in the colonization of the lung by strains IMT5155 and IMT5155Δ*yqi* as seen in figure 5B with a *p*<0.02 [19]. The average lung score in chickens infected with IMT5155Δ*yqi* was found to be 1.52 in comparison to 2.4 and 0.6 in chickens infected with IMT5155 and IMT11327 respectively. In both infection models, the ability of IMT11327 to colonize the chicken lung was much less than both IMT5155 and IMT5155Δ*yqi* as seen in figure 5.

### Effect of EA/I during systemic infection in chickens

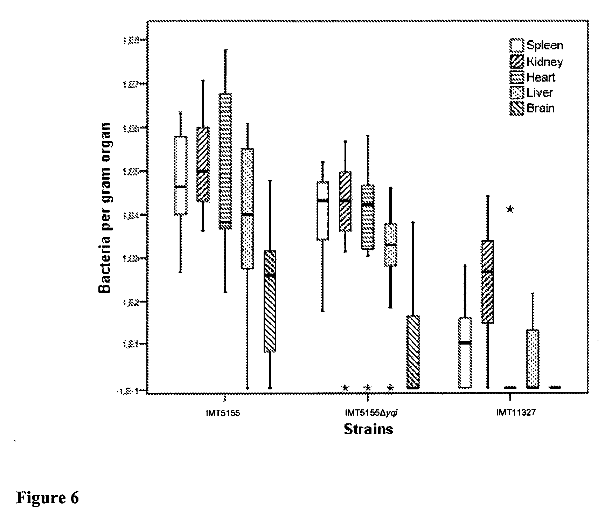

Systemic infection can be induced in chickens by infecting 5-week old birds intra-tracheally with an infection dose of 10⁹ CFU. As mentioned before, infection of chickens with this infection dose resulted in significant reduction in bacterial colonization of the chicken lung. When bacteria were re-isolated from chicken internal organs including spleen, kidneys, heart, liver and brain, there was a reduction in bacterial numbers when infected with IMT5155Δypi as compared to IMT5155 as seen in figure 6, however only marginal. The only exception was the brain, where almost no bacteria were isolated when infected with IMT5155Δ*yqi* as compared to IMT5155. All organ scores were considerably reduced in chickens infected with IMT5155Δ*yqi* as compared to chickens infected with IMT5155 as seen in table 4.

### Electron microscopy reveals fimbrial like appendages associated with ExPEC adhesin I (yqi) gene cluster

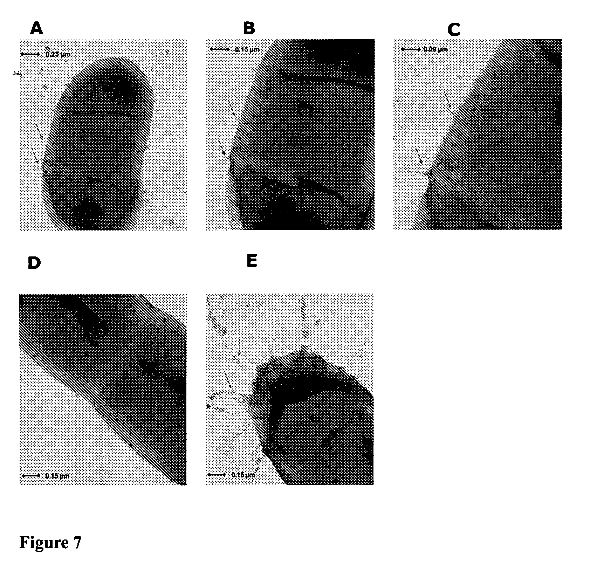

The ExPEC adhesin I (*yqi*) 4975bp gene cluster coding for the putative subunit, chaperone, usher and adhesin was cloned and over-expressed in an afimbriate strain *E. coli* AAEC189 using a suitable expression vector. Negative staining of strain AAEC189 (pKESK:*yqi*_4975_XB) revealed the expression of short fimbrial like appendages forming on the outer membrane of the bacterial cell (Figure 7). These appendages were about 0.04 µm long and 0.005 µm thick and were not detected in the afimbriate strain AAEC189, that is, the negative control (Figure 8A). The wild type strain IMT5155, harbouring other adhesins like Type 1 fimbriae, curli and temperature-sensitive haemagglutin, in addition to ExPEC adhesin I, was used as a positive control for the staining method, and long fimbriae with a length of about 0.5 µm (Figure 8B) were observed which were morphologically different from the fimbrial structures observed in strain AAEC189 (pKESK:*yqi*_4975_XB).

### Prevalence of EA/I among ExPEC reveals its potential as a factor of virulence

A collection of ExPEC strains, intestinal pathogenic *E. coli* and avian commensals available at the Institute of Microbiology and Epizootics, Freie Universität Berlin, was screened to determine the presence of the *yqi* gene among these strains. Out of a total of 588 ExPEC isolates tested for the presence of *yqi*, including 406 APEC, 138 UPEC, 25 NMEC and 19 SePEC, 368 isolates were found to be positive for *yqi*, which amounts to a total of 62.5% of pathogenic isolates harbouring the *yqi* gene (Table 3). Among APEC isolates alone, 221 isolates were found to be positive making it a total of 54.4% positive for *yqi* in this group. Among UPEC isolates 91 isolates were found to be positive in a total percentage of 65.9% positive for *yqi* and among NMEC isolates 15 were found to be positive amounting to 60.0% positive for *yqi*. Finally, among SePEC isolates, 10 were positive in a percentage of 52.6%. From a total of 159 A_{faecal} isolates tested, 31 were found to be positive *for yqi,* which accounts for only 19.4% of the non pathogenic strains tested.
Results were compared with MLST/EcoR data deposited in the publicly available database (www.mist.net), of which 292 of the pathogenic isolates tested were known for their sequence type (ST) and 607 were known for their EcoR groups according to Herzer et al (Herzer PJ, Inouye S, Inouye M, Whittam TS (1990) Phylogenetic distribution of branched RNA-linked multicopy single-stranded DNA among natural isolates of Escherichia coli. J Bacteriol 172: 6175-6181.), which was carried out as part of a separate study at the Institute of Microbiology and Epizootics. Of the isolates positive for *yqi,* 68 isolates belonged to the EcoR group A (18.9%), 4 to B1 (1.1%), 255 to B2 (70.8%) and 33 to D (9.1%) making B2 the predominant group for isolates harbouring *yqi*. Among isolates negative for *yqi,* 122 isolates belonged to EcoR group A (49.3%), 29 to B1 (11.7%), 42 to B2 (17.0%) and 54 to D (21.8%) where A is the predominant group for isolates lacking *yqi*.
When comparing the distribution of the *yqi* gene among ExPEC strains with available MLST data, it was observed that strains positive for *yqi* are mostly allotted to sequence types 12, 68, 73, 95, 104, 140, 141, 349, 355, 358, 372, 390, 420, 913. All strains belonging to sequence types 141, 372 and sequence type complex 95 were positive for *yqi.*
Among 153 intestinal pathogenic *E. coli* tested in this study, none of the isolates were found to be positive for *yqi* as seen in table 3.
Due to the exclusive association of the putative fimbrial adhesin gene *yqi* with extraintestinal pathogenic *E. coli* (ExPEC), that is, its high prevalence among ExPEC isolates compared with non pathogenic *E. coli,* and complete absence in intestinal pathogenic *E. coli,* the adhesin was temporarily designated ExPEC adhesin I (EA/I).

### EA/I is evolving under positive selection

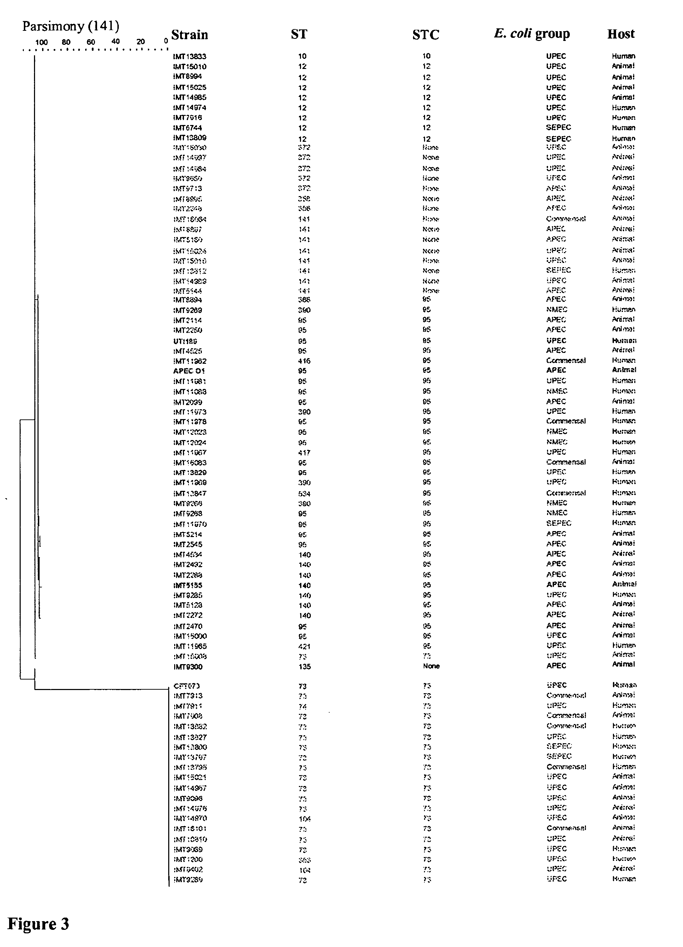

The ExPEC adhesin I gene (*yqi*) was sequenced in many strains and compared with existing Multilocus sequence typing (MLST) data. MLST is a nucleotide sequence based approach for the unambiguous characterization of isolates of bacteria using sequences of internal fragments of seven house-keeping genes. The population structure of microbial species with intermediate levels of recombination can thus be revealed by allele-based analyses. Wirth et al. described that sequence polymorphisms could define unique sequences for each of the seven house-keeping gene loci, which are referred to as alleles and each unique combination of alleles is assigned a sequence type (ST) number. Related STs are assigned to so-called ST complexes, using the principles of the eBurst algorithm: each ST complex includes at least three STs that differ from their nearest neighbour by no more than two of the seven loci while ST complexes differ from each other by three or more loci, and STs that did not match the criteria for inclusion within an ST complex are simply referred to by their ST designation.
Sequencing of the ExPEC adhesin I (*yqi*) gene in strains belonging to the various sequence types (ST) and sequence type complexes (STC) including STC10, STC12, STC73, STC95, ST141 and ST372, and calculation of the distances of the adhesin gene locus between these strains revealed distribution of the strains tested into two groups (Figure 3). One group included strains belonging to the ST73 complex, while all other strains were assigned to a second group. Sequence homology was observed among strains belonging to a particular sequence type or sequence type complex as is the case with ST141, ST372, STC12 and STC73 within the sequence type or sequence type complex (Figure 3). One exception to the rule was strain IMT15008, ST73, STC73 which showed variations in its *yqi* gene sequence compared to other strains in the ST73 complex, and could be better assigned to the group harbouring other strains tested. Interestingly, only strains belonging to sequence type complex 95 showed mutations in the *yqi* gene sequence. Therefore, we determined the ratio of the non-synonymous mutation rate (Dn) to the synonymous mutation rate (Ds) within this complex using DnaSP 4.50.3 software. Dn/Ds < or =1 indicates purifying or neutral selection, favouring amino acid substitutions. Our data show a non-synonymous mutation rate of 0.00095496 and a synonymous mutation rate of 0.00024 resulting in a Dn/Ds ratio of 3.979, indicating strong positive selection for structural evolution of the adhesin gene *yqi* within the sequence type 95 complex.

### Discussion

A necessary step in the successful colonization and progression of disease by microbial pathogens is the ability to adhere to host surfaces. The present study made use of a lung colonization chicken infection model in order to identify APEC genes specific to early stages of infection including adhesion to and colonization of the host by its pathogen via signature-tagged mutagenesis. As anticipated, we identified fimbrial proteins including the type 1 fimbrial regulatory protein, and other novel genes explicitly involved in the early stages of APEC pathogenesis as determined by competition assays with mutants. Our most interesting finding was a novel *yqi* adhesin which was seen to play a very significant role in the colonization of the avian lung by APEC. The newly identified putative fimbrial adhesin encoding the gene *yqi,* will be temporarily called ExPEC adhesin I (EA/I) until the specific host receptor for this adhesin has been identified in order to allow better classification and nomenclature of the novel *E. coli* fimbrial adhesin.
Adhesins are known to facilitate host colonization by mediating the first and crucial interaction with host tissue. In certain cases pathogens have been reported to have a substantial amount of different adhesins expressed at one time or another. In *E. coli,* two of the best characterized adhesins are the type 1 fimbriae and the P fimbriae which are regulated by the *fim* and pap operons. Genome sequencing of prototypic cystitis UPEC strain UT189 has now revealed ten different chaperone-usher adhesin systems, among which the *auf, yad, yfc, yqi, yeh* and *fml* operons still remain to be characterized (Chen SL, Hung CS, Xu J, Reigstad CS, Magrini V, et al. (2006) Identification of genes subject to positive selection in uropathogenic strains of Escherichia coli: a comparative genomics approach. Proc Natl Acad Sci U S A 103: 5977-5982.).
Interestingly, we identified the UPEC *yqi* adhesin gene in an STM screen specially designed to detect factors that would play a crucial role in the initiation of APEC infection. As stated before, the APEC *yqi* gene showed complete sequence identity with the UPEC *yqi* and is part of a ∼5kb adhesin gene cluster yet uncharacterized. When comparing this adhesin gene sequence with publicly available *E. coli* genomes, the adhesin was found to be present among ExPEC strains including APEC and UPEC and is not harboured by non-pathogenic *E*. *coli* nor by any of the intestinal pathogenic *E. coli* like Enteropathogenic *E. coli* (EPEC) or Enterohaemorrhagic *E. coli* (EHEC), making it unique to extraintestinal infections. This result therefore validates the temporary name ExPEC adhesin I (EAII) allotted to the new adhesin. Fimbrial adhesins share common genetic organization, in that the adhesin regulatory genes precede the major subunit gene, which is followed by the periplasmic chaperone, outer membrane usher, and finally the adhesin genes. This gene cluster organization is seen in type 1 fimbriae *(fim),* S fimbriae (sfa), F1C fimbriae (*foc*) and the Dr-antigen recognizing fimbriae (*dra*). Organization of the *yqi* adhesin gene cluster differs by having the positions of the usher and chaperone inverted, which is also true for the pap adhesin gene cluster of the P fimbriae, although the reason for this is still unclear.
To date, there are no studies implying the role of ExPEC adhesin I (*yqi*) in the colonization of host tissues during infection. Previously, it has been shown that the *fimH* adhesin of the type 1 fimbriae, and the *papG* adhesin of the P fimbriae are the receptor specific binding proteins that are located at the tip of the pilus structure. It has also been reported that deletion of the *papG* gene had no effect on pilus formation; however, the pili isolated from a *papG* deletion strain were not adhesive. We now have evidence which shows that deletion of the EA/I gene, *yqi,* in APEC strain IMT5155 results in a significant decrease in the colonization of fibroblasts and epithelial cells *in vitro* and of the chicken lung *in vivo.* It is possible that the *yqi* adhesin is the receptor-specific protein responsible for initial attachment to host cells; however, this receptor is yet to be identified.
APEC strain IMT5155 has been previously tested for the presence of important adhesins like *fim, pap, crl, sfa, tsh, afa, dra, foc* and others, and has been found to harbour only the type 1 fimbria *(fim),* curli *(crl)* and temperature-sensitive haemagglutinin *(tsh)* genes (Ewers C, Li G, Wilking H, Kiessling S, Alt K, et al. (2007) Avian pathogenic, uropathogenic, and newborn meningitis-causing Escherichia coli: How closely related are they? Int J Med Microbiol 297: 163-176.). The absence of P, F1C, S and Dr fimbriae in IMT5155, which are known for their role in UPEC pathogenesis, made it apparent that there might in fact be unidentified adhesins that participate crucially during initiation of infection. We observed that in both infection models used in this study, there was always a substantial reduction in the colonization of the chicken lung when infecting chickens with IMT5155Δ*yqi*. Therefore, we believe that ExPEC adhesin I makes a significant contribution to total bacterial adhesion and colonization of the chicken lung by APEC strain IMT5155 during infection.
An interesting result was the prevalence of EA/I among a relatively large collection of strains, particularly its high incidence among ExPEC strains like APEC, UPEC, NMEC and septicaemia associated *E. coli* (SEPEC) strains, infrequent occurrence among non-pathogenic strains and complete absence among intestinal pathogenic *E. coli* strains. It has been previously reported that distinctive strains of *E. coli* responsible for most cases of urinary tract infection, sepsis and newborn meningitis are derived predominantly from *E. coli* phylogenetic group B2. Additionally, diverse studies show that virulent clonal groups are derived mainly from phylogenetic group B2 and to a lesser extent from group D. Our observations show that 66.1% of the isolates positive for *yqi* belong to the phylogenetic group B2 in contrast to only 11.5% of isolates negative for *yqi* belonging to this group.
Furthermore, we found that all of the isolates belonging to sequence types 95, 140, 141 and 372 were positive for *yqi.* ST95 and ST140 belong to the sequence type 95 complex. It is known that well defined pathogens are associated with specific STs or ST complexes, for example, ST95 complex contains related pathogenic bacteria of serogroups O1, 02, and O18 that express the K1 polysaccharide, that is, the K1 isolates. Of immense interest, therefore, was the evolutionary analysis of the adhesin gene sequences from strains belonging to different sequence types, which showed that within a particular sequence type complex (STC), the adhesin sequence was homologous which is the case for STC12 and STC73 as well as other sequence types like ST372, ST141 and ST358 with a single exception, strain IMT15008. Since this strain had no unique characteristic that differentiated it from other strains within the ST73 complex, a possible explanation could be that the *yqi* gene in strain IMT15008 is the result of a recombination event.
Interestingly, within the ST95 complex, the adhesin gene sequence showed the presence of single nucleotide polymorphisms (SNPs) which were confirmed as a positive selection on the gene within this complex. Mutations producing functional modifications are called pathogenicity-adaptive or pathoadaptive, and are often SNPs producing amino acid replacements in proteins essential for a pathogen's success and it has been shown that two major adhesins of extraintestinal pathogenic E.coli (ExPEC) - type 1 and P fimbrial adhesins - acquire structural SNPs at a rapid rate, and this adaptation constitutes a major factor in the pathoadaptive microevolution and genetic diversification of ExPEC clonal groups. It is possible, that ExPEC adhesin I also undergoes structural mutations or pathoadaptive mutations, particularly, among strains belonging to the ST95 complex, which further confirms the importance of this adhesin within this highly pathogenic complex. Not surprisingly therefore, is the fact that the wild type strain used in this study, IMT5155, also belongs to the ST 95 complex and harbours the most number of SNPs in the gene coding for ExPEC adhesin I.
It therefore makes sense to assume that ExPEC adhesin I could perhaps play a very specific role in ExPEC pathogenesis, especially during the early colonization steps of infection. However, an interesting addition to this story is the decrease in adhesion to kidney epithelial cells as shown by our *in vitro* adhesion experiments. This would indicate the importance of *yqi* in UPEC, which particularly colonize the urinary tract during urinary tract infection (UTI). Furthermore, it provides evidence for the zoonotic potential of APEC, a topic of great interest in the field of ExPEC.
The presence of a single virulence factor hardly ever makes a strain virulent, while a combination of virulence factors usually determines its ability to cause disease. Therefore, much like countless other genes that are classified into the class of virulence factors when exceedingly prevalent among pathogens, the *yqi* gene could also eventually be an addition to this category based on its regular presence in highly pathogenic ExPEC strains in contrast to non pathogenic strains. One must keep in mind, that the meager group of A_{faecal} strains that were found to be positive for *yqi* in this study are no classical non-pathogenic strains as seen in a previous study (Ewers C, Antao EM, Diehl I, Philipp HC, Wieler LH (2009) Intestine and environment of the chicken as reservoirs for extraintestinal pathogenic Escherichia coli strains with zoonotic potential. Appl Environ Microbiol 75: 184-192.). Ewers et al reported that a number of A_{faecal} *E. coli* strains have characteristics typical of human and animal ExPEC, and that some nonoutbreak strains are capable of causing systemic disease in immunocompetent 5-week old chickens, suggesting the avian intestine reservoir hypothesis. Furthermore it was reported that these strains pose a zoonotic risk because they could be transferred directly from birds to humans or serve as a genetic pool for ExPEC strains. Therefore, taken together, the results of the ExPEC adhesin I (*yqi*) prevalence study in *E. coli* are in fact intriguing, since the search for novel virulence factors still goes on.
An interesting result in this study was the successful expression of ExPEC adhesin *I in vitro.* We hypothesized that cloning of the putative adhesin gene cluster coding for the putative subunit protein, putative usher and chaperone proteins and putative adhesin, which together are believed to be responsible for expression of fimbrial structures, when successfully cloned in an afimbriate *E. coli* strain would enable the expression of EA/1 fimbriae visible under the electron microscope. Using negative staining and transmission electron microscopy, our hypothesis was indeed confirmed, in that, short fimbrial like structures were detected in the afimbriate strain overexpressed with the adhesin gene cluster, which were not observed in the afimbriate strain, or negative control. The wild type strain IMT5155 used as a positive control revealed long fimbrial structures representing all fimbrial adhesins harboured by the strain including ExPEC adhesin I which explains the difference in length of fimbrial structures observed. The short fimbrial structures observed in the afimbriate strain overexpressed with ExPEC adhesin I will have to be confirmed using specific antibody and immunogold staining in the future; however, this study provides preliminary evidence for the fimbrial structures of this newly identified fimbrial adhesin. ExPEC adhesin I is therefore confirmed to be a novel fimbrial adhesin.
We have identified an adhesin that plays a significant role in the initial stages of APEC infection. This suggests that antibodies elicited against this adhesin can impede colonization, block infection and prevent disease, that is, prophylactic vaccination with said new adhesin can inhibit bacterial infections. Blocking initial stages of infection may be one of the most effective strategies to prevent bacterial infections.

**Table 1. Strains and plasmids used in this study.**

| **Strain** | **Description** |
|---|---|
| IMT5155 | O2:K1:H5; *csgA, fimC, tsh, chuA, fyuA, ireA, iroN, irp2, iucD, iutA, sit, neuC, ibeA, gimB, colV, ompA,* ST140, STC95, B2 |
| IMT5155 Nal^{R} | IMT5155 derivative, Nalidixin resistant |
| S17λpir | *recA thi* pro *hsdR-* M+ RP4::2-Tc::Mu::Km Tn7 lysogenized with λpir phage |
| IMT11327 | *Ont:Hl6*; *fimC, crlA, ompA,* ST 295, B1 |
| MG1655 | F⁻ ,Lam⁻, Fim⁺ |
| EA7F9 | IMT5155 Nal^{R} *yqi*::mini-Tn5 |
| IMT5155Δ*yqi* | IMT5155 *Nat^{R} Δyqi::Cm^{R}* |
| *E. coli* TOP10 | F- *mcrA Δ(mrr-hsdRMS-mcrBC)* Φ80lacZΔM15 *ΔlacX74 recA1 araD139* Δ(araleu)7697 *galU galK rpsL* (StrR) *endA1 nupG* |
| pCR2.1-TOPO:*yqi* | *E. coli* TOP10 [pCR2.1 TOPO:*yqi*] |
| pDSK602:*yqi* | *E. coli* TOP10 [pDSK602:*yqi*] |
| IMT5155Δ*yqi* (pDSK602:*yqi*) | IMT5155 Nal^{R} *Δyqi*::Cm^{R} [pDSK602:*yqi*] |
| IMTS155Δ*yqi* (pDSK602) | IMT5155 Nal^{R} *Δyqi::Cm^{R}* [pDSK602] |
| IMT5155 (pDSK602-*yqi*) | IMT5155 Nal^{R} [pDSK602:*yqi*] |
| pCR2.1-TOPO:*yqi*_operon | *E. coli* TOP10 [pCR2.1TOPO:*yqi*-5kb] |
| Top10 (pKESK:*yqi*_4975_XB) | *E.coli* TOP10 [pKESK-22:*yqi*_4975bp_Xbal-BamHI] |
| *E. coli* AAEC189 | Δfim, Δlac, recA-, endA-, hsdR-, hsdM+ |
| *E. coli* AAEC189 | *E.coli* AAEC189 [pKESK-22:*yqi*_4975bp:XbaI-BamHI] |
| (pKESK:*yqi*_4975_XB) | |
| **Plasmid** | |
| pUTmini-Tn5km2 | Kan^{R}, Amp^{R} |
| pKD46 | Amp^{R}, expresses λ red recombinase |
| pKD3 | *cat* gene |
| pCR2.1 TOPO | Kan^{R}, Amp^{R}, LacZα, T7 promoter |
| pKESK-22 | Neo^{R}, Kan^{R}, *tac* promoter |
| pDSK602 | Spec^{R}, Sm^{R}, triple *lac* UV5, broad host range |

**Table 2. Oligonucleotide primers used in this study.**

| **Primer number** | **Target region** | **Primer Sequence (5'-3')** | **Tm (°C)** | **SEQ ID NO** |
|---|---|---|---|---|
| IMT2510 | *cat* (pKD3) | | 75 | 16 |
| IMT2511 | *cat* (pKD3) | | 71 | 17 |
| IMT2512 | *yqi* | ATGCAATGGCAGTACCCTTC | 60 | 4 |
| IMT2513 | *yqi* | CTGGTGGCAACATCAAATTG | 60 | 5 |
| IMT2558 | 221 bp upstream of *yqi* | ATTACCGTCGGTTATATCGGC | 52 | 6 |
| IMT2559 | 110 bp downstream of *yqi* | ATAAACACAATATGGCGCTCG | 50 | 7 |
| IMT2910 | *yqi* with *EcoRI* | CGGATACGAATTCATGATTACGCTTTTTCGTT | 59 | 8 |
| IMT2911 | *yqi* with *HindIII* | TTCTCAAAAGCTTTGTCGTTCAGTTATAGTTTA | 57 | 9 |
| IMT3045 | *yqi* operon with *BamHI* | | 56 | 10 |
| IMT3046 | *yqi* operon with *HindIII* | | 53 | 11 |
| IMT1560 | Cloning vector | GTAAAACGACGGCCAG | | 18 |
| | pCR2.1 TOPO (-20) | | 50 | |
| IMT1561 | Cloning vector | CAGGAAACAGCTATGAC | | 19 |
| | pCR2.1 TOPO | | 50 | |
| IMT718 | *Cat* | TTATACGCAAGGCGACAAGG | 57.3 | 20 |
| IMT719 | *Cat* | GATCTTCCGTCACAGGTAGG | 59.4 | 21 |
| IMT3138 | Expression vector pKESK-22 | AATGTGTGGAATTGTGAGCGG | 60.6 | 22 |
| IMT3139 | Expression vector pKESK-22 | GCCGACATGATCCAACTGA | 60.1 | 23 |
| IMT3706 | *yqi* | AGTTAGGCTTTGTGGCGGAC | 56.3 | 12 |
| IMT3707 | *yqi* | GTTCACCGTCTATCTCCT | 53.6 | 13 |
| IMT3259 | *yqi* operon with *XbaI* | | 67.3 | 14 |
| IMT3260 | *yqi* operon with *BamHI* | | 66.6 | 15 |

**Table 3. Prevalence of ExPEC adhesin I coding gene yqi among pathogenic E. coli strains and non-pathogenic A_{faecal} strains**

| ***E. coli* Group** | **Isolates tested** | **Prevalence of *yqi* (%)** |
|---|---|---|
| APEC | 406 | 54.4 |
| | | |
| UPEC | 138 | 65.9 |
| | | |
| NMEC | 25 | 60.0 |
| | | |
| SePEC | 19 | 52.6 |
| | | |
| STEC | 49 | 0.0 |
| | | |
| EHEC | 46 | 0.0 |
| | | |
| EPEC | 28 | 0.0 |
| | | |
| aEPEC | 12 | 0.0 |
| | | |
| ETEC | 8 | 0.0 |
| | | |
| EIEC | 6 | 0.0 |
| | | |
| EAEC | 4 | 0.0 |
| | | |
| Commensals/ (A_{faecal}) isolates | 159 | 19.4 |

| | | |
|---|---|---|
| APEC=Avian pathogenic *E. coli,* UPEC=Uropathogenic *E. coli,* NMEC= Newborn meningitic *E*. *coli,* SePEC=Septicaemia associated *E. coli,* STEC=Shiga Toxin-producing *E. coli*, EHEC=Enterohaemorrhagic *E. coli,* EPEC=Enteropathogenic *E. coli,* aEPEC=Atypical Enteropathogenic *E. coli,* ETEC= Enterotoxigenic E. coli, EIEC=Enteroinvasive *E. coli,* EAEC=Enteroggregative *E. coli.* | | |

**Table 4. Score values for severity of organ lesions ± standard deviation in respiratory and other organs.**

| **Strain** | **Average Organ Score (Infection dose: 10⁹ CFU)** | | | | |
|---|---|---|---|---|---|
| | Air sacs | Lung | Liver | Heart | Spleen |
| **IMT5155** | 2.0 ± 1.0 | 2.4 ± 0.9 | 0.8 ± 0.8 | 1.8 ± 1.3 | 1.0 ± 0.0 |
| **IMT5155Δ*yqi*** | 1.1 ± 0.7* | 1.5 ± 0.6* | 0.1 ± 0.3* | 0.7 ± 0.8 | 0.9 ± 0.3 |
| **IMT11327** | 1.6 ± 0.5* | 2.0 ± 0.5* | 0.0 ± 0.0* | 0.0 ± 0.0 | 0.9 ± 0.5 |

| | | | | | |
|---|---|---|---|---|---|
| Infection with IMT5155, IMT5155Δyqi and IMT11327 at infection dose 10⁹ CFU. Differences in organ scores between IMT5155 and IMT5155Δyqi and between IMT5155 and negative control IMT11327 were statistically significant for air sacs, lungs and liver with a *p*<0.05*. | | | | | |

## Claims

1. Isolated nucleic acid for use in treatment or diagnosis of infectious diseases, wherein the nucleic acid comprises or consists of:
- the nucleic acid sequence of the *yqi* gene with SEQ ID NO. 1 or the reverse complement thereof;
- a nucleic acid sequence with at least 95% sequence identity to SEQ ID NO. 1 or a reverse complement thereof;
- a nucleic acid sequence hybridizing under stringent conditions to the nucleic acid sequence with SEQ ID NO. 1 or to its reverse complement;
**characterized in that**
the infectious disease is a disease associated with infection of a vertebrate with extraintestinal pathogenic APEC bacteria.

2. Nucleic acid of claim 1, wherein the nucleic acid comprises or consists of:
- the nucleic acid sequence of the *yqi* gene cluster with SEQ ID NO. 2 or the reverse complement thereof;
- a nucleic acid sequence with at least 95% sequence identity to SEQ ID NO. 2 or a reverse complement thereof; or
- a nucleic acid sequence hybridizing under stringent conditions to the nucleic acid sequence with SEQ ID NO. 2 or to its reverse complement.

3. Nucleic acid of claim 1, wherein the nucleic acid comprises or consists of a nucleic acid sequence selected from SEQ ID NO. 1, 2, 4 to 15 or a reverse complement thereof.

4. Isolated polypeptide for use in treatment or diagnosis of infectious diseases, wherein the polypeptide comprises or consists of an amino acid sequence encoded by a nucleic acid of one of claims 1 to 3, preferably by the nucleic acid sequence of the *yqi* gene with SEQ ID NO. 1 or by a nucleic acid sequence with at least 95% sequence identity to SEQ ID NO. 1; **characterized in that**
the infectious disease is a disease associated with infection of a vertebrate with extraintestinal pathogenic APEC bacteria.

5. Polypeptide of claim 4, wherein the polypeptide comprises or consists of the amino acid sequence with SEQ ID NO. 3.

6. Antibody being specific for a polypeptide of claim 4 or 5 for use in treatment or diagnosis of infectious diseases, **characterized in that** the infectious disease is a disease associated with infection of a vertebrate with extraintestinal pathogenic APEC bacteria

7. Nucleic acid and/or polypeptide of claims 1 to 6 for use in diagnosis of an infectious disease, wherein the diagnosis comprises the steps of:
- providing a probe;
- contacting the probe with a binder being specific for a nucleic acid of claim 1, 2 or 3 and/or a polypepide of claim 4 or 5 under conditions allowing for specific binding of the binder to said nucleic acid or polypeptide; and
- detection of the binder being specifically bound to the nucleic acid of claim 1, 2 or 3 and/or the polypeptide of claim 4 or 5.

8. Nucleic acid and/or polypeptide of claim 7, wherein the binder is a nucleic acid hybridizing under stringent conditions to a nucleic acid of claim 1, 2 or 3, an aptamer or an antibody of claim 6 or 7.

9. Use of a nucleic acid of claim 1, 2 or 3 and/or a polypeptide of claim 4 or 5 in the manufacture of a vaccine for use in prophylaxis of a vertebrate against infection with APEC bacteria.

## Patentansprüche

1. Isolierte Nucleinsäure zur Verwendung bei der Behandlung oder Diagnose von Infektionskrankheiten, wobei die Nucleinsäure
- die Nucleinsäuresequenz des yqi-Gens mit SEQ ID NO: 1 oder deren reverses Komplement,
- eine Nucleinsäuresequenz mit wenigstens 95% Sequenzidentität zu SEQ ID NO: 1 oder ein reverses Komplement derselben,
- eine Nucleinsäuresequenz, die unter stringenten Bedingungen an die Nucleinsäuresequenz mit SEQ ID NO: 1 oder an deren reverses Komplement hybridisiert,
umfasst oder daraus besteht,
**dadurch gekennzeichnet, dass**
es sich bei der Infektionskrankheit um eine Erkrankung handelt, die mit einer Infektion eines Wirbeltiers mit extraintestinalen pathogenen APEC-Bakterien verbunden ist.

2. Nucleinsäure nach Anspruch 1, wobei die Nucleinsäure
- die Nucleinsäuresequenz des yqi-Genclusters mit SEQ ID NO: 2 oder deren reverses Komplement,
- eine Nucleinsäuresequenz mit wenigstens 95% Sequenzidentität zu SEQ ID NO: 2 oder ein reverses Komplement derselben, oder
- eine Nucleinsäuresequenz, die unter stringenten Bedingungen an die Nucleinsäuresequenz mit SEQ ID NO: 2 oder an deren reverses Komplement hybridisiert,
umfasst oder daraus besteht.

3. Nucleinsäure nach Anspruch 1, wobei die Nucleinsäure eine Nucleinsäuresequenz, die ausgewählt ist aus SEQ ID NO: 1, 2, 4 bis 15 oder einem reversen Komplement derselben, umfasst oder daraus besteht.

4. Isoliertes Polypeptid zur Verwendung bei der Behandlung oder Diagnose von Infektionskrankheiten, wobei das Polypeptid eine Aminosäuresequenz umfasst oder aus einer Aminosäuresequenz besteht, die von einer Nucleinsäure nach einem der Ansprüche 1 bis 3 codiert wird, vorzugsweise von der Nucleinsäuresequenz des yqi-Gens mit SEQ ID NO: 1 oder von einer Nucleinsäuresequenz mit wenigstens 95% Sequenzidentität zu SEQ ID NO: 1.
**dadurch gekennzeichnet, dass**
es sich bei der Infektionskrankheit um eine Erkrankung handelt, die mit einer Infektion eines Wirbeltiers mit extraintestinalen pathogenen APEC-Bakterien verbunden ist.

5. Polypeptid nach Anspruch 4, wobei das Polypeptid die Aminosäuresequenz mit SEQ ID NO: 3 umfasst oder daraus besteht.

6. Antikörper, der spezifisch für ein Polypeptid nach Anspruch 4 oder 5 ist, zur Verwendung bei der Behandlung oder Diagnose von Infektionskrankheiten, **dadurch gekennzeichnet, dass** es sich bei der Infektionskrankheit um eine Erkrankung handelt, die mit einer Infektion eines Wirbeltiers mit extraintestinalen pathogenen APEC-Bakterien verbunden ist.

7. Nucleinsäure und/oder Polypeptid nach den Ansprüchen 1 bis 6 zur Verwendung bei der Diagnose einer Infektionskrankheit, wobei die Diagnose die folgenden Schritte umfasst:
- Bereitstellen einer Sonde;
- Zusammenbringen der Sonde mit einem für eine Nucleinsäure nach den Ansprüchen 1, 2 oder 3 und/oder für ein Polypeptid nach Anspruch 4 oder 5 spezifischen Binder unter Bedingungen, die spezifische Bindung des Binders an die Nucleinsäure oder das Polypeptid ermöglichen, und
- Nachweisen des Binders, der spezifisch an die Nucleinsäure nach den Ansprüchen 1, 2 oder 3 und/oder das Polypeptid nach Anspruch 4 oder 5 gebunden ist.

8. Nucleinsäure und/oder Polypeptid nach Anspruch 7, wobei der Binder eine Nucleinsäure, die unter stringenten Bedingungen an eine Nucleinsäure nach den Ansprüchen 1, 2 oder 3 hybridisiert, ein Aptamer oder ein Antikörper nach Anspruch 6 oder 7 ist.

9. Verwendung einer Nucleinsäure nach den Ansprüchen 1, 2 oder 3 und/oder eines Polypeptids nach Anspruch 4 oder 5 bei der Herstellung eines Impfstoffs zur Verwendung bei der Prophylaxe eines Wirbeltiers gegen Infektion mit APEC-Bakterien.

## Revendications

1. Acide nucléique isolé destiné à une utilisation dans le traitement ou le diagnostic des maladies infectieuses, dans lequel l'acide nucléique comprend ou consiste en :
- la séquence d'acide nucléique du gène *yqi* correspondant au SEQ ID n° 1 ou le complément inverse de celle-ci,
- une séquence d'acide nucléique présentant au moins 95 % d'identité de séquence avec le SEQ ID n° 1 ou un complément inverse de celle-ci,
- une séquence d'acide nucléique s'hybridant dans des conditions stringentes à la séquence d'acide nucléique correspondant au SEQ ID n° 1 ou à son complément inverse,
**caractérisé en ce que**
la maladie infectieuse est une maladie associée à une infection d'un vertébré avec une bactérie APEC pathogène extra-intestinale.

2. Acide nucléique selon la revendication 1, dans lequel l'acide nucléique comprend ou consiste en :
- la séquence d'acide nucléique du groupe de gènes *yqi* correspondant au SEQ ID n° 2 ou le complément inverse de celle-ci,
- une séquence d'acide nucléique présentant au moins 95 % d'identité de séquence avec le SEQ ID n° 2 ou un complément inverse de celle-ci, ou
- une séquence d'acide nucléique s'hybridant dans des conditions stringentes à la séquence d'acide nucléique correspondant au SEQ ID n° 2 ou à son complément inverse.

3. Acide nucléique selon la revendication 1, dans lequel l'acide nucléique comprend ou consiste en une séquence d'acide nucléique sélectionnée parmi le groupe des SEQ ID n° 1, 2, 4 à 15 ou un complément inverse d'entre elles.

4. Polypeptide isolé destiné à une utilisation dans le traitement ou le diagnostic des maladies infectieuses, dans lequel le polypeptide comprend ou consiste en une séquence d'acides aminés codée par un acide nucléique selon l'une des revendications 1 à 3, de préférence par la séquence d'acide nucléique du gène *yqi* correspondant au SEQ ID n° 1 ou par une séquence d'acide nucléique présentant au moins 95% d'identité de séquence avec le SEQ ID n° 1,
**caractérisé en ce que**
la maladie infectieuse est une maladie associée à une infection d'un vertébré avec une bactérie APEC pathogène extra-intestinale.

5. Polypeptide selon la revendication 4, dans lequel le polypeptide comprend ou consiste en la séquence d'acides aminés correspondant au SEQ ID n° 3.

6. Anticorps étant spécifique à un polypeptide selon la revendication 4 ou 5, destiné à une utilisation dans le traitement ou le diagnostic de maladies infectieuses, **caractérisé en ce que** la maladie infectieuse est une maladie associée à une infection d'un vertébré avec une bactérie pathogène APEC extra-intestinale.

7. Acide nucléique et/ou polypeptide selon les revendications 1 à 6 destiné à une utilisation dans le diagnostic d'une maladie infectieuse, dans lequel le diagnostic comprend les étapes consistant à :
- prévoir une sonde,
- mettre en contact la sonde avec un ligand étant spécifique pour un acide nucléique selon la revendication 1, 2 ou 3 et/ou un polypeptide selon la revendication 4 ou 5 dans des conditions permettant une liaison spécifique du ligand au dit acide nucléique ou polypeptide, et
- détecter le ligand étant lié spécifiquement à l'acide nucléique selon la revendication 1, 2 ou 3 et/ou au polypeptide selon la revendication 4 ou 5.

8. Acide nucléique et/ou polypeptide selon la revendication 7, dans lequel le ligand est un acide nucléique s'hybridant dans des conditions stringentes à un acide nucléique selon la revendication 1, 2 ou 3, un aptamère ou un anticorps selon la revendication 6 ou 7.

9. Utilisation d'un acide nucléique selon la revendication 1, 2 ou 3 et/ou d'un polypeptide selon la revendication 4 ou 5 dans la préparation d'un vaccin destiné à être utilisé dans la prophylaxie d'un vertébré contre une infection avec une bactérie APEC.
